# EUROPEAN PATENT SPECIFICATION

(11) **EP 2 788 491 B1**
(45) Date of publication and mention of the grant of the patent: **23.01.2019**
(21) Application number: 12806777.4
(22) Date of filing: 06.12.2012
(51) Int. Cl.: C12N 15/75

(54) **RIBOSOMAL PROMOTORS FROM B. SUBTILIS FOR PROTEIN PRODUCTION IN MICROORGANISMS**
RIBOSOMALE PROMOTOREN AUS B. SUBTILIS ZUR PROTEINPRODUKTION IN MIKROORGANISMEN
PROMOTEURS RIBOSOMAUX ISSUS DE B. SUBTILIS POUR LA PRODUCTION DE PROTÉINES DANS DES MICROORGANISMES

(30) Priority: 09.12.2011 US 201161569202 P; 19.12.2011 US 201161577491 P
(43) Date of publication of application: 15.10.2014
(73) Proprietor: Danisco US Inc., Palo Alto, CA 94304 (US)
(72) Inventor: BONGIORNI, Cristina, Palo Alto, CA 94304 (US); FOX, Bryan P., Palo Alto, CA 94304 (US); VAN KIMMENADE, Anita, Palo Alto, CA 94304 (US)
(74) Representative: Mewburn Ellis LLP
(86) International application number: PCT/US2012/068285
(87) International publication number: WO 2013/086219

(56) References cited:
- WO-A2-2008/148575
- NIJLAND ET AL: "Heterologous production and secretion of Clostridium perfringens beta-toxoid in closely related Gram-positive hosts", JOURNAL OF BIOTECHNOLOGY, ELSEVIER SCIENCE PUBLISHERS, AMSTERDAM, NL, vol. 127, no. 3, 1 December 2006 (2006-12-01), pages 361-372, XP005787028, ISSN: 0168-1656, DOI: 10.1016/J.JBIOTEC.2006.07.014
- KRASNY LIBOR ET AL: "An alternative strategy for bacterial ribosome synthesis: Bacillus subtilis rRNA transcription regulation", EMBO (EUROPEAN MOLECULAR BIOLOGY ORGANIZATION) JOURNAL, vol. 23, no. 22, 10 November 2004 (2004-11-10), pages 4473-4483, XP9167931, ISSN: 0261-4189 cited in the application
- STEWART G C ET AL: "DNA sequence of the tandem ribosomal RNA promoter for B. subtilis operon rrnB.", NUCLEIC ACIDS RESEARCH 24 SEP 1983, vol. 11, no. 18, 24 September 1983 (1983-09-24), pages 6289-6300, XP002693775, ISSN: 0305-1048
- LUKACSOVICH T ET AL: "A FAMILY OF EXPRESSION VECTORS BASED ON THE RRNB P2 PROMOTER OF ESCHERICHIA COLI", JOURNAL OF BIOTECHNOLOGY, ELSEVIER SCIENCE PUBLISHERS, AMSTERDAM, NL, vol. 16, no. 1 / 02, 1 October 1990 (1990-10-01), pages 49-55, XP000150708, ISSN: 0168-1656, DOI: 10.1016/0168-1656(90)90064-I

## Description

### FIELD OF THE INVENTION

The present invention relates to the production of proteins in microorganisms. In particular, the present invention provides methods and compositions of improved expression systems in microorganisms. In certain embodiments, the methods and compositions comprise a ribosomal promoter derived from a *Bacillus* species microorganism.

### BACKGROUND OF THE INVENTION

Genetic engineering has allowed the improvement of microorganisms used as industrial bioreactors or cell factories. For example, *Bacillus* species produce and secrete a large number of useful proteins and metabolites. The most common *Bacillus* species used in industry are *B. licheniformis, B. amyloliquefaciens,* and *B. subtilis.* Because of their GRAS (generally recognized as safe) status, strains of these *Bacillus* species are natural candidates for the production of proteins utilized in the food and pharmaceutical industries. Important production enzymes include α-amylases, neutral proteases, and alkaline (or serine) proteases. However, in spite of advances in the understanding of production of proteins in *Bacillus* host cells, there remains a need for methods to improve the expression and production of these proteins by microorganisms.

Recombinant production of a product encoded by a gene is accomplished by constructing expression vectors suitable for use in a host cell in which the nucleic acid coding for a desired product is placed under the expression control of a promoter. The expression vector is introduced into a host cell by various techniques, such as transformation, and production of the desired product is then achieved by culturing the transformed host cell under suitable conditions necessary for the functioning of the promoter included in the expression vector. While numerous promoters are known in the art, there is a need for new promoters, which improve the expression of heterologous genes and coding sequences.

The regulation of ribosomal RNA transcription from ribosomal RNA promoters has been studied previously (Krasny and Gourse in The EMBO Journal (2004) 23, 4473-4483).

### SUMMARY OF THE INVENTION

The present invention provides novel nucleic acids, expression vectors, microorganisms, and methods for the production of a protein of interest. In particular, the present invention provides novel nucleic acids, expression vectors, microorganisms, and methods for the production of a protein of interest comprising *Bacillus subtilis* ribosomal RNA promoter. The protein of interest is selected from the group consisting of a protease, cellulase, amylase, xylanase, phytase, mannanase, hemicellulase, carbohydrase, hydrolase, esterase, oxidase, permease, pullulanase, laccase, lipase, reductase, isomerase, epimerase, tautomerase, transferase, kinase or phosphatase. The ribosomal RNA promoter comprises the nucleotide sequence of any one of SEQ ID NOs: 2&3, a nucleic acid that is a subsequence having at least 80% sequence identity to any one of SEQ ID NOs: 2&3 that retains promoter activity, or combinations thereof.

In one embodiment, the invention provides a nucleic acid comprising a B. subtilis ribosomal promoter operably linked to a nucleic acid encoding a protein of interest as defined in claim 1.

In another embodiment, the invention provides an expression vector comprising a nucleic acid of

In another embodiment, the invention provides a microorganism comprising an expression vector of the invention.

In another embodiment, the invention provides a method for producing a protein of interest comprising culturing a microorganism of the invention under conditions suitable for the to produce the protein. The methods may comprise the step of recovering the protein of interest thus produced.

Also provided is a method for producing a protein of interest without amplification of an expression construct. The method may comprises transforming a microorganism with a nucleic acid or vector comprising a ribosomal promoter, wherein the nucleic acid or vector integrates into the host cell as a single integrant, and culturing the microorganism under conditions suitable for the microorganism to produce the protein. The ribosomal promoter is a ribosomal RNA promoter.

Also provided is a method of producing a protein of interest by introducing a nucleic acid or vector described herein into a host cell so that it integrates into the host cell but does not require the use of an antibiotic marker.

The ribosomal RNA promoter is a rrn promoter derived from B. subtilis, in particualr an rrnI ribosomal RNA promoter.

In a specific embodiment, the ribosomal RNA promoter is a P2 rrnI ribosomal RNA promoter from B. subtilis.

The ribosomal RNA promoter comprises the nucleotide sequence of any one of SEQ ID NOs: 2&3, or a nucleic acid that is a subsequence having at least 80% sequence identity to any one of SEQ ID NOs: 2&3 that retains promoter activity.

In a specific embodiment, the ribosomal RNA promoter comprises the nucleotide sequence of SEQ ID NO: 3. In other embodiments, combinations of any of the above promoters can be used. For example, one or more of a P1 or P2 promoter of a rrnI promoter can be used together.

The ribosomal promoters described herein can be operably linked to a nucleic acid encoding a protein of interest. The protein of interest is an enzyme selected from a protease, cellulase, amylase, xylanase, phytase, mannanase, hemicellulase, carbohydrase, hydrolase, esterase, oxidase, permease, pullulanase, laccase, lipase, reductase, isomerase, epimerase, tautomerase, transferase, kinase, and phosphatase. In a particular embodiment, the protein of interest is a protease. In another particular embodiment the protein of interest is a subtilisin. In a specific embodiment, the protein of interest is encoded by SEQ ID NOs: 9, 11, 18 or 20.

A protein of interest can be heterologous or homologous to the microorganism in which it is expressed. In certain embodiments, the nucleic acid, vector, or expression construct that is used to express the nucleic acid encoding the protein of interest is integrated into the host cell. In certain embodiments, the nucleic acid, vector, or expression construct that is used to express the nucleic acid encoding the protein of interest is not integrated into the host cell. The nucleic acid, vector, or expression construct that is used to express the nucleic acid encoding the protein of interest can be amplified in the host cell or it can be maintained as a single copy.

Any bacterial or fungal microorganism that is capable of expression from a ribosomal promoter can be used herein as a host cell. In certain embodiments, the microorganism is a gram positive microorganism. In some embodiments, the microorganism is a member of the genus Bacillus. Examples of Bacillus cells that are useful in the invention include, for example, B. subtilis, B. licheniformis, B. lentus, B. brevis, B. stearothermophilus, B. alkalophilus, B. amyloliquefaciens, B. coagulans, B. circulans, B. lautus, and B. thuringiensis. In other embodiments, the microorganism is E. coli, Pseudomonas spp. (e.g., P. aeruginoa and P. alcaligenes), or Streptomyces spp., (e.g., Streptomyces lividans).

### BRIEF DESCRIPTION OF THE DRAWINGS

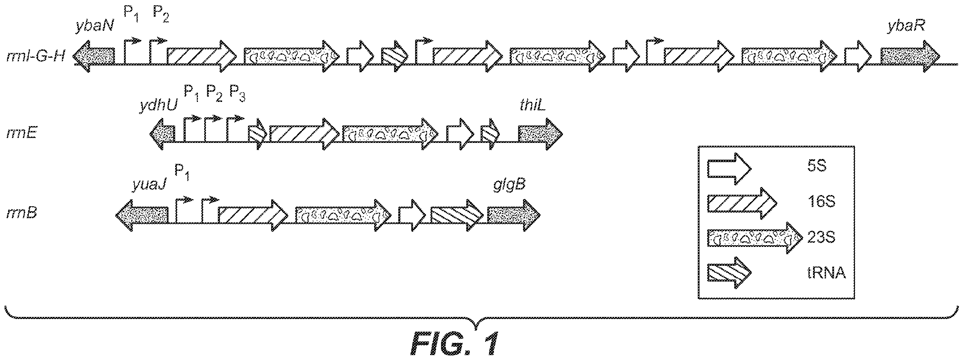

Figure 1 shows organization of the *Bacillus subtilis rrn* operons used in this study. The different strains constructed from the fusion of the promoters to the target genes are listed in Table 1-2.
Figure 2 shows the alignment of rrnE P2 with the P1 promoters from rrnA, rrnB, rrnI, rrnD, rrnE, rrnJ, and rrnO. Figure 2 also shows the -35 and -10 regions of each promoter, as well as the upstream "UP" elements for each promoter that are upstream of the -35 sequence of each promoter.
Figure 3 shows the alignment of the rrnE P3 promoter with the P2 promoter from rrnA, rrnB, rrnI, rrnW, rrnH, rrnG, rrnD, rrnJ, and rrnO. Figure 3 also shows the -35 and -10 regions of each promoter, as well as the upstream "UP" elements for each promoter that are upstream of the -35 sequence of each promoter.
Figure 4 is a graph showing the cell density measurements for strains expressing GFP from various Papre, PrrnI, PrrnE or PrrnB promoters.
Figure 5 is a graph showing the cell density measurements for strains expressing FNA from Papre, PrrnI promoters in strain BG8000.
Figure 6 is a graph showing the cell density measurements for strains expressing FNA from Papre, PrrnI, PrrnE, and PrrnB promoters in strain BG8010.

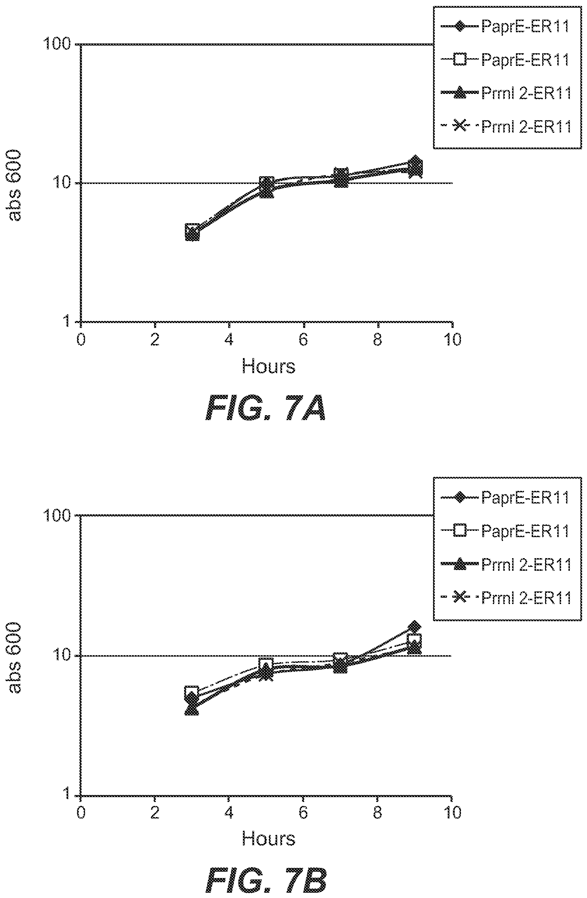

Figures 7A and 7B are graphs showing the cell density measurements for strains expressing ER11 from PaprE and PrrnI promoters in strains BG8000 and BG8010.
Figure 8 is a graph showing GFP expression from PaprE, PrrnI, PrrnE, and PrrnB promoters.
Figure 9 is a graph showing FNA expression from PaprE and PrrnI promoters.
Figure 10 is a graph showing FNA expression from PaprE, PrrnI, PrrnE, and PrrnB promoters.

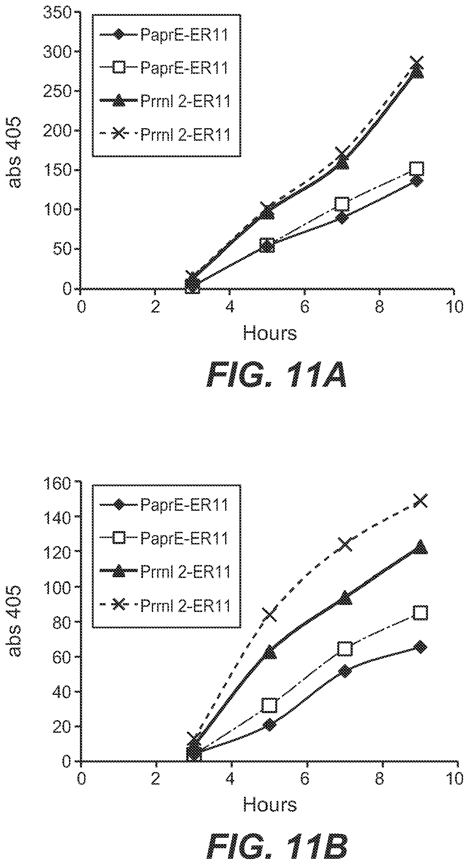

Figure 11 is a graph showing ER11 expression from PaprE and PrrnI promoters.
Figure 12 is a graph showing cell density measurements of strains expressing FNA from P*aprE* and P*rrnI* promoters.
Figure 13 is a graph showing strains expressing FNA from P*aprE* and P*rrnI* promoters.
Figure 14 is a graph showing cell density measurements of FNA expression from single copy integrants of P*aprE* and P*rrnI* promoter constructs.
Figure 15 is a graph showing FNA expression from FNA expression from single copy integrants of P*aprE* and P*rrnI* promoter constructs.

### DETAILED DESCRIPTION OF THE INVENTION

The present invention provides improved methods and compositions for expression systems in microorganisms. More specifically, the methods and compositions comprise a ribosomal promoter derived from a *Bacillus* species microorganism, more specifically ribosomal RNA promoters. In some embodiments, novel production microorganisms and methods for producing a protein of interest are provided.

### A. Definitions

Unless defined otherwise herein, all technical and scientific terms used herein have the same meaning as commonly understood by one of ordinary skill in the art to which this invention belongs (See e.g., Singleton, et al., DICTIONARY OF MICROBIOLOGY AND MOLECULAR BIOLOGY, 2D ED., John Wiley and Sons, New York [1994], and Hale & Marham, THE HARPER COLLINS DICTIONARY OF BIOLOGY, Harper Perennial, NY [1991]). Although any methods and materials similar or equivalent to those described herein can be used in the practice or testing of the present invention, the preferred methods and materials are described.

Numeric ranges are inclusive of the numbers defining the range. Unless otherwise indicated, nucleic acids are written left to right in 5' to 3' orientation; amino acid sequences are written left to right in amino to carboxy orientation, respectively.

The headings provided herein are not limitations of the various aspects or embodiments of the invention. Accordingly, the terms defined immediately below are more fully defined by reference to the specification as a whole.

As used herein, the term "nucleic acid sequence" encompasses DNA, RNA, single or doubled stranded and modification thereof. The terms "nucleic acid sequence" and "polynucleotide" may be used interchangeability herein.

As used herein, "polypeptide," "peptide" and "protein" are used interchangeably and include reference to a polymer of amino acid residues. The terms apply to amino acid polymers in which one or more amino acid residue is an artificial chemical analog of a corresponding naturally occurring amino acid, as well as to naturally occurring amino acid polymers. The terms also apply to polymers containing conservative amino acid substitutions such that the polypeptide remains functional.

As used herein, the term "host cell" refers to a cell that has the capacity to act as a host and expression vehicle for an incoming sequence (i.e., a sequence introduced into the cell), as described herein. In one embodiment, the host cell is a microorganism. In a preferred embodiment, the host cells are Bacillus species.

As used herein, "Bacillus" refers to all species, subspecies, strains and other taxonomic groups within the genus Bacillus, including, but not limited to B. subtilis, B. licheniformis, B. lentus, B. brevis, B. stearothermophilus, B. alcalophilus, B. amyloliquefaciens, B. coagulans, B. circulans, B. lautus, and B. thuringiensis.

As used herein, the term "DNA construct" or "expression construct" refers to a nucleic acid sequence, which comprises at least two DNA polynucleotide fragments. A DNA or expression construct can be used to introduce nucleic acid sequences into a host cell or organism. The DNA may be generated in vitro (e.g., by PCR) or any other suitable techniques. In some preferred embodiments, the DNA construct comprises a sequence of interest. The sequence of interest's nucleic acid is operably linked to a promoter. In some embodiments, the DNA construct further comprises at least one selectable marker. In further embodiments, the DNA construct comprises sequences homologous to the host cell chromosome. In other embodiments, the DNA construct includes non-homologous sequences.

As used herein, the terms "nucleic acid encoding a protein of interest" or "coding sequence of interest" are used interchangeably and mean a nucleic acid sequence that encodes a protein of interest when translated into the protein. In some embodiments, the coding region is present in a cDNA form, while in other embodiments, it is present in genomic DNA or RNA form. When present in a DNA form, the oligonucleotide may be single-stranded (i.e., the sense strand) or double-stranded. In some embodiments, suitable control elements (e.g., enhancers, promoters, splice junctions, polyadenylation signals, etc.) are placed in close proximity to the coding region of the gene if needed to permit proper initiation of transcription and/or correct processing of the primary RNA transcript. Alternatively, in some embodiments, the coding region utilized in the expression vectors of the present invention contain endogenous enhancers, splice junctions, intervening sequences, polyadenylation signals, or a combination of both endogenous and exogenous control elements.

As used herein, the terms "promoter," "promoter element," and "promoter sequence," refer to a DNA sequence which is capable of controlling the transcription of an oligonucleotide sequence into mRNA when the promoter is placed at the 5' end of (i.e., precedes) an oligonucleotide sequence. Thus, a promoter is typically located 5' (i.e., upstream) of an oligonucleotide sequence whose transcription into mRNA it controls, and provides a site for specific binding by RNA polymerase and for initiation of transcription. As used herein a ribosomal promoter includes, for example, a ribosomal RNA promoter or a ribosomal protein promoter.

The term "operably linked" refers to juxtaposition, wherein elements are in an arrangement allowing them to be functionally related. For example, a promoter is operably linked to a coding sequence of interest if it controls the transcription of the sequence.

As used herein, the term "promoter activity" when made in reference to a nucleic acid sequence refers to the ability of the nucleic acid sequence to initiate transcription of an oligonucleotide sequence into mRNA.

The term "vector" is defined herein as a polynucleotide designed to carry nucleic acid sequences to be introduced into one or more cell types. Vectors include cloning vectors, expression vectors, shuttle vectors, plasmids, phage or virus particles, DNA constructs, cassettes and the like. Typical expression vectors, which also include plasmids, include regulatory sequences such as promoters, signal sequences, a gene of interest and transcription terminators.

The term "isolated" as defined herein, refers to a compound, protein, cell, nucleic acid sequence, or amino acid that is separated from at least one other compound, protein, cell, nucleic acid sequence, amino acid, or other biological substance with which it is ordinarily associated in its natural source.

As used herein the term "coding region" is defined herein as a nucleic acid sequence that is transcribed into mRNA which is translated into a polypeptide when placed under the control of appropriate control sequences including a promoter. A coding sequence may include cDNA, genomic DNA, synthetic DNA and recombinant DNA.

As used herein, the term "wild-type" gene, gene product, or cell refers to a gene, gene product, or cell which has the characteristics of that gene, gene product, or cell when found in a naturally occurring source. A wild-type gene, gene product, or cell is that which is most frequently observed in a population and is thus designated the "normal" or "wild-type" form. As used herein, the terms "wild-type sequence," and "wild-type gene" are used interchangeably and refer to a sequence that is native or naturally occurring in a host cell.

In contrast, the term "modified," "mutant," or "variant" gene, gene product, or cell refers to a gene, gene product, or cell which displays modifications in sequence and/or functional properties (i.e., altered characteristics) when compared to the wild-type form. Sequence modifications can occur by, for example, substitutions, insertions, deletions, or any other modification that results in an altered sequence or characteristic. It is noted that naturally-occurring mutants can be isolated; these are identified by the fact that they have altered characteristics when compared to the wild-type gene or gene product.

As used herein, the terms "modified sequence" and "modified genes" are used interchangeably and refer to a substitution, insertion, deletion, interruption, or any other modification of naturally occurring nucleic acid sequence. In some embodiments, the expression product of the modified sequence is a truncated protein (e.g., if the modification is a deletion or interruption of the sequence). In some embodiments, the truncated protein retains biological activity. In other embodiments, the expression product of the modified sequence is an elongated protein (e.g., if the modification is an insertion into the nucleic acid sequence). In other embodiments, an insertion results in the production of a truncated protein as the expression product (e.g., if the insertion results in the formation of a stop codon).

As used herein, an "incoming sequence" means a DNA sequence that is introduced into the host cell chromosome or genome. The sequence may encode one or more proteins of interest. The incoming sequence may comprise a promoter operably linked to a sequence encoding a protein of interest. In some embodiments, incoming sequences comprise sequence that is already present in the genome of the cell to be transformed, while in other embodiments, it is not already present in the genome of the cell to be transformed (i.e., in some embodiments, it is homologous, while in other embodiments, it is heterologous sequence).

In some embodiments, the incoming sequence encodes at least one homologous or heterologous protein, including, but not limited to a hormone, enzyme, growth factor, or cytokine. In some preferred embodiments, the incoming sequence encodes at least one enzyme including, but not limited to a protease, cellulase, amylase, xylanase, phytase, mannanase, hemicellulase, carbohydrase, hydrolase, esterase, oxidase (such as phenol oxidase), permease, pullulanase, laccase, lipase, reductase, isomerase, epimerase, tautomerase, transferase, kinase, or phosphatase.

In some embodiments, the incoming sequence encodes a functional wild-type gene or operon, a functional mutant gene or operon, or a non-functional gene or operon.

As used herein, the term "reporter gene" refers to a nucleotide sequence, which is capable of expression in cells and where expression of the reporter confers to cells containing the expressed gene, the ability to be easily detected and measured.

As used herein, the term "flanking sequence," refers to any sequence that is either upstream or downstream of the sequence being discussed (e.g., for sequences A B C, sequence B is flanked by the A and C sequences). In some embodiments, the incoming sequence is flanked by a homology box on each side.

As used herein, the term "homology box" refers to sequences that are homologous to another nucleic acid sequence. For example, a homology box can be homologous to a nucleic acid sequence in genomic DNA. In such instance, the homology box is useful for directing where in a new construct is integrated into the genomic DNA.

As used herein, the term "homologous recombination" refers to the exchange of DNA fragments between two DNA molecules or paired chromosomes (i.e., during crossing over) at the site of identical nucleotide sequences. In one embodiment, chromosomal integration is accomplished via homologous recombination.

As used herein, the term "heterologous" in general refers to a polynucleotide or polypeptide that does not naturally occur in a host cell, or refers to a polynucleotide or polypeptide that is derived from the same genetic source or species as the host cell, but is in a location that is not native to the heterologous sequence. In some embodiments, a heterologous sequence is a non-host sequence, while in other embodiments, it is a modified sequence, a sequence from a different host cell strain, or a homologous sequence from a different chromosomal location of the host cell.

The terms "transfection" and "transformation" as used herein both refer to methods for introducing DNA into cells.

As used herein, the terms "complementary" or "complementarity" are used in reference to "polynucleotides" and "oligonucleotides" (which are interchangeable terms that refer to a sequence of nucleotides) related by the base-pairing rules. For example, the sequence "5'-CAGT-3'," is complementary to the sequence "5'-ACTG-3'." Complementarity can be "partial" or "total." "Partial" complementarity is where one or more nucleic acid bases is not matched according to the base pairing rules. "Total" or "complete" complementarity between nucleic acids is where each and every nucleic acid base is matched with another base under the base pairing rules.

As used herein, the term "chromosomal integration" refers to the process whereby the incoming sequence is introduced into the chromosome (i.e., genome) of a host cell.

As used herein, the term "selectable marker" refers to the use of any "marker" (i.e., indicator), which indicates the presence or absence of a protein or gene of interest. In some embodiments, the term encompasses genes which encode an enzymatic activity that confers the ability to grow in medium lacking what would otherwise be essential. In other embodiments, a selectable marker confers resistance to an antibiotic or drug upon the cell in which the selectable marker is expressed.

As used herein, the term "signal sequence" or "signal peptide" refers to a sequence of amino acids at the N-terminal portion of a protein, which facilitates the secretion of the mature form of the protein outside the cell. The mature form of the extracellular protein lacks the signal sequence which is cleaved off during the secretion process.

"Amplification" is defined herein as the production of additional copies of a nucleic acid sequence. Amplification of a nucleic acid can be performed by, for example, polymerase chain reaction or other technologies that are well known in the art. As used herein, the term "polymerase chain reaction" ("PCR") refers to the methods of U.S. Pat. Nos. 4,683,195, 4,683,202, and 4,965,188 which describe a method for increasing the concentration of a segment of a target sequence in a DNA sample (e.g., genomic DNA) without cloning or purification.

With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; or incorporation of .sup.32P-labeled deoxynucleotide triphosphates, such as dCTP or DATP, into the amplified segment). In addition to genomic DNA, any oligonucleotide sequence can be amplified with the appropriate set of primer molecules. In particular, the amplified segments created by the PCR process itself are, themselves, efficient templates for subsequent PCR amplifications.

As used herein, the term "primer" refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced. The primer is preferably single stranded for maximum efficiency in amplification, but may alternatively be double stranded.

As used herein, the term "probe" refers to an oligonucleotide, whether occurring naturally as in a purified restriction digest or produced synthetically, which is capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that it is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, the terms "restriction endonucleases" and "restriction enzymes" refer to bacterial enzymes, each of which cut double- or single-stranded DNA at or near a specific nucleotide sequence.

### B. Ribosomal Promoters

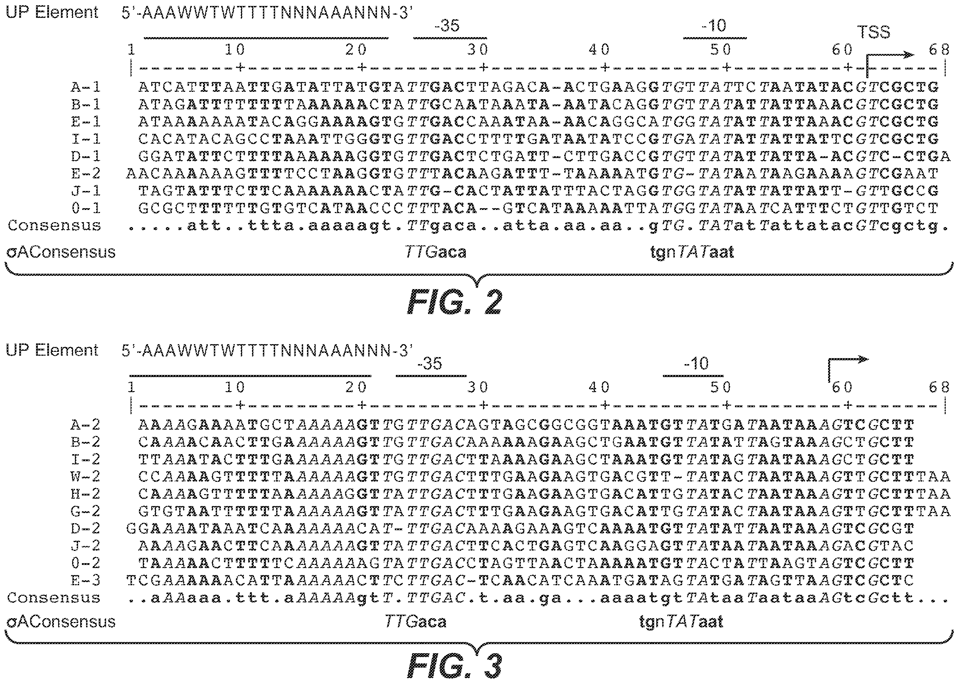

Ribosomal RNA synthesis is the rate-limiting step in ribosome synthesis in *Escherichia coli* and *Bacillus subtilis.* The regulation of ribosomal RNA transcription from ribosomal RNA promoters has been studied previously (Samarrai et al., 2011, J Bacteriology, 193:723-733; Natori et al., 2009, J Bacteriology, 191:4555-4561; Turnbough, 2008, Molecular Microbiology 69:10-14; Krasny et al., 2008, Mol Microbiology 69:42-54; Krasny and Gourse, 2004, EMBO 23:4473-4483;). rRNA promoters are tightly regulated with nutritional conditions so that ribosomal RNA and ribosomes are not overproduced in times when translational requirements are lower. In *E. coli,* there are seven rRNA (rrn) operons, each of which contains two promoters designated P1 and P2. The core -10/-35 region in E. *coli* rrn P1 promoters is preceded by upstream (UP) elements that increase promoter activity by up to 20-50 fold by binding RNA polymerase. *Bacillus subtilis,* contains 10 rrn operons (Krasny and Gourse, *supra*), which are also preceded by upstream (UP) elements that can help to increase promoter activity. See Figures 2 and 3.

Although the regulation of ribosomal RNA promoters has been studied for the production of native ribosomal RNAs, the expression levels of a nucleic acid sequence coding for a heterologous protein of interest when using ribosomal RNA promoters has never been investigated.

The regulation of the genes that encode ribosomal proteins has been studied previously in *Escherichia coli* and *Bacillus subtilis* (Grundy and Henkin, 1991, J. Bacteriology, 173:4595-4602) In many cases, the ribosomal proteins have been found to act as an autogenous repressor, controlling the expression of the operon in which they are encoded.

The present disclosure demonstrates that ribosomal promoters, such as ribosomal RNA and protein promoters, are unexpectedly effective at producing heterologous proteins of interest. The amount of mRNA transcribed from a ribosomal promoter was surprisingly high both when compared to other commonly used promoters and as measured by the number of mRNA molecules produced per unit time. See, for example, Examples 3-5 which compare expression from ribosomal promoters to the highly expressed apre promoter. In one embodiment, the ribosomal promoter of the invention provides enhanced transcription efficiency as measured by the number of mRNA molecules produced per unit of time.

The unexpectedly high level of expression of a nucleic acid sequence coding for a heterologous protein of interest when using ribosomal promoters has several benefits. In one embodiment, expressing a coding sequence of interest with a ribosomal promoter allows for increased level of expression of a coding sequence of interest when compared to expression of the coding sequence of interest from its native promoter. An increased level of expression is particularly useful for transcripts that are unstable.

In another embodiment, expressing a coding sequence of interest with a ribosomal promoter allows for increased level of expression of a coding sequence of interest without amplification of an expression construct comprising the ribosomal promoter. When using other expression constructs in the art, in order to achieve high expression levels of a coding sequence of interest, amplification of the expression construct is often required. The expression levels achieved with the ribosomal promoters described herein, however, are high enough that amplification of the expression construct is not necessary. Instead, high expression levels are achieved with a single integrant of the expression construct comprising the ribosomal promoter. See Examples 4 and 5. This provides several benefits. First, host strains are more stable because they do not undergo the loss of the amplified expression construct. Also, if an expression construct does not need to be amplified, strain construction is more efficient. Thus, time, money and materials are saved.

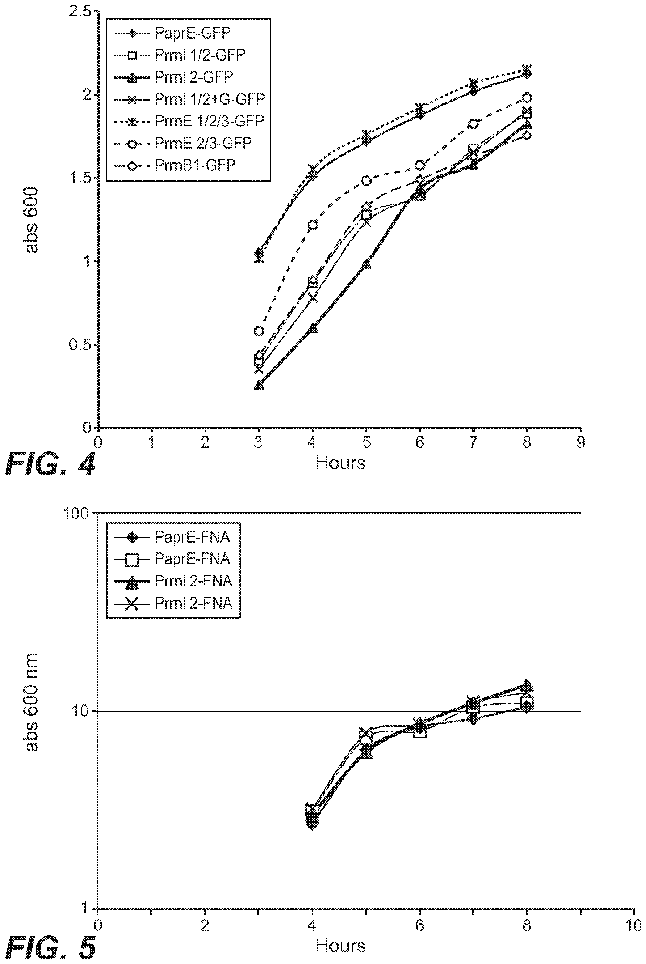

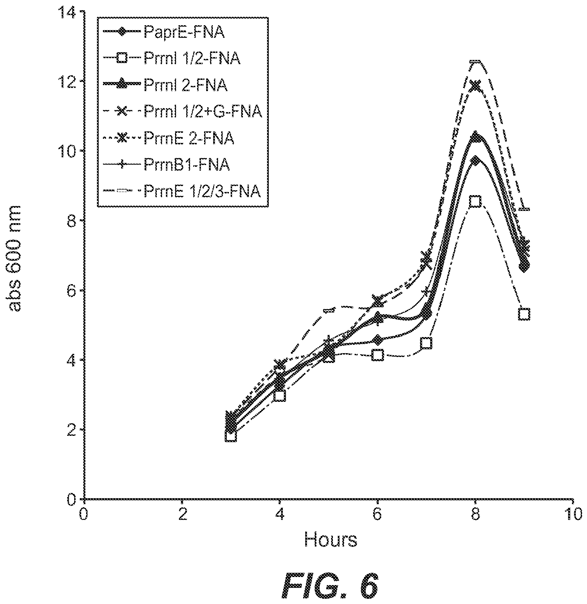

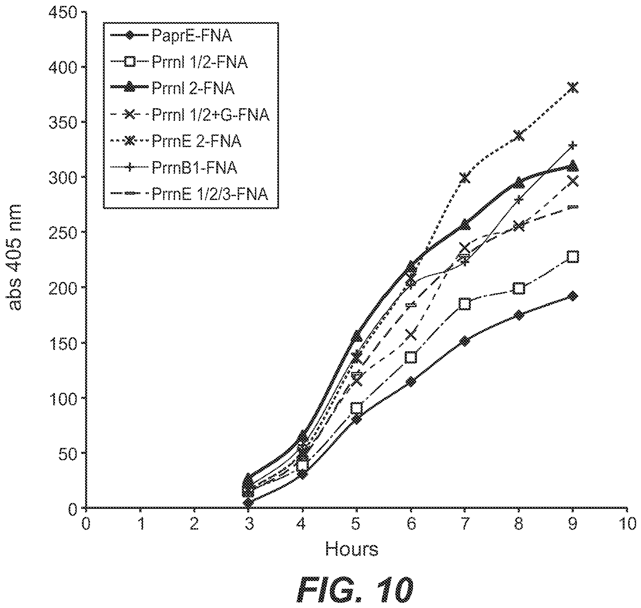

In some embodiments, the ribosomal promoters are ribosomal RNA promoters. The ribosomal RNA promoters used in the invention may be any one of the P1 or P2 promoters from a Bacillus rrnI ribosomal RNA promoter. In one embodiment, the RNA promoter used in the invention is the P2 promoter from a Bacillus rrnI ribosomal RNA promoter. In some embodiments, combinations of the P1 & P2 promoters from a Bacillus rrnI ribosomal RNA promoter can be used. See, for example, Examples 2-4 and Figures 4, 8, 6, and 10.

In a particular embodiment, the nucleotide located at the +1 transcriptional start site of a ribosomal promoter (e.g., a ribosomal RNA or protein promoter) described herein is modified from a guanine to adenine. For example, the transcriptional start site for the ribosomal RNA promoters described herein is shown in Figures 2 and 3. Modification of the +1 transcriptional start site allows consistent production from a promoter described herein, and therefore, results in better overall productivity from the promoter.

In one embodiment, a promoter has the nucleic acid sequence of any one of SEQ ID NOs: 2&3, a nucleic acid that is a subsequence having at least 80% sequence identity to any one of SEQ ID NOs: 2&3 that retains promoter activity or combinations thereof. The subsequence comprises at least
about 60 nucleotides; at least about 70 nucleotides; at least about 80 nucleotides; at least about 90 nucleotides or at least about 100 nucleotides. The subsequence of any one of SEQ ID NOs: 2 and 3 may minimally comprise the -35 and -10 regions of the parent promoter. For example, the subsequence of any one of SEQ ID NOs: 2 and 3 may minimally comprise the -35 and -10 regions of the parent promoter as illustrated in Figures 2 and 3, or Tables 1-1. In certain embodiments, a subsequence of any of SEQ ID NOs: 2 and 3 may comprise the -35 and -10 regions of the parent promoter and further comprises the upstream UP elements of the parent promoter, as illustrated in Figures 2 and 3.

In a particular embodiment, the promoter has the nucleic acid sequence of SEQ ID NO: 3.

The promoter may also be a tandem promoter, which comprises two or more promoters. In some embodiments, the tandem promoter will include the promoter of any one of SEQ ID NOs: 2 and 3 may or a subsequence thereof having at least 80% sequence identity to any one of SEQ ID NOs: 2&3 that retains promoter activity.

A tandem promoter, a promoter which is a subsequence of any one of SEQ ID NOs: 2 and 3 may have at least about 20%, at least about 30%, at least about 40%, least about 50%, at least about 60%, at least about 80%, and at least about 100% of the promoter activity of its corresponding parent promoter. In some embodiments, the promoter activity will be greater, for example more than about 100%, more than about 150%, more than about 200% and more than about 250%.

In a particular embodiment, hybridization is used to analyze whether a given DNA fragment corresponds to a promoter DNA sequence described herein and thus falls within the scope of the present invention. Sambrook et al., MOLECULAR CLONING: A LABORATORY MANUAL (2.sup.nd Ed., 1989 Cold Spring Harbor, N.Y.) describes general hybridization methods.

"Hybridization conditions" refers to the degree of "stringency" of the conditions under which hybridization is measured. Hybridization conditions can be based on the melting temperature (Tm) of the nucleic acid binding complex, as taught in Berger and Kimmel (1987, Guide to Molecular Cloning Techniques, METHODS IN ENZYMOLOGY, Vol 152, Academic Press, San Diego Calif.). Hybridization conditions can also be based on the washing conditions employed after hybridization as known in the art.

Merely for purposes of illustration, "Low-stringency" conditions can refer to washing with a solution of 0.2x SSC/0.1% SDS at 20 C for 15 minutes. "Medium-stringency" conditions can refer to washing with a solution of 0.2x SSC/0.1% SDS at 37 C for 30 minutes. "High-stringency" conditions can refer to washing with a solution of 0.2x SSC/0.1% SDS at 37 C for 45 minutes. "Very high-stringency" conditions can refer to washing with a solution of 0.2x SSC/0.1% SDS at 37 C for 60 minutes. However, the stringency associated with the particular solution ingredients, temperature, and wash time can vary depending on the particular nucleic acids and other conditions involved. The skilled person would be able to determine the hybridization conditions associated with a desired degree of stringency.

Another aspect of the invention is use of hybridization conditions based on the melting temperature (Tm) of the nucleic acid binding complex, as taught in Berger and Kimmel (1987, Guide to Molecular Cloning Techniques, METHODS IN ENZYMOLOGY, Vol. 152, Academic Press, San Diego, Calif. For purposes of illustration, "very high stringency" typically occurs at about Tm-5 C (5 C below the Tm of the probe); "high stringency" typically occurs at about 5 C to 10 C below Tm; "medium stringency" at about 10 C to 20 C below Tm; and "low stringency" at about 20 C to 25 C below Tm.

One example of a hybridization assay may be performed as follows: Genomic DNA from a particular target source is fragmented by digestion with an appropriate restriction enzyme, e.g., EcoR I, Hind III, Bam HI, Cla I, Kpn I, Mlu I, Spe I, Bgl II, Nco I, Xba I, Xho I and Xma I (supplied by New England Biolabs, Inc., Beverly, Mass. and Boehringer Mannheim) according to the manufacturer's instructions. The samples are then electrophoresed through an agarose gel (for example, 0.8% agarose) so that separation of DNA fragments can be visualized by size. DNA fragments are typically visualized by ethidium bromide staining. The gel may be briefly rinsed in distilled H₂O and subsequently depurinated in an appropriate solution (such as, for example, 0.25M HCl) with gentle shaking followed by denaturation for 30 minutes (in, for example, 0.4 M NaOH) with gentle shaking. A renaturation step may be included, in which the gel is placed in 1.5 M NaCl, 1M Tris, pH 7.0 with gentle shaking for 30 minutes. The DNA should then be transferred onto an appropriate positively charged membrane, for example, Maximum Strength Nytran Plus membrane (Schleicher & Schuell, Keene, N.H.), using a transfer solution (such as, for example, 6x SSC (900 mM NaCl, 90 mM trisodium citrate). Once the transfer is complete, generally after about 2 hours, the membrane is rinsed in e.g., 2x SSC (2x SSC=300 mM NaCl, 30 mM trisodium citrate) and air dried at room temperature. The membrane should then be prehybridized (for approximately 2 hours or more) in a suitable prehybridization solution (such as, for example, an aqueous solution containing per 100 mL: 20-50 mL formamide, 25 mL of 20x SSPE (1x SSPE=0.18 M NaCl, 1 mM EDTA, 10 mM NaH₂PO₄, pH 7.7), 2.5 mL of 20% SDS, and 1 mL of 10 mg/mL sheared herring sperm DNA). As would be known to one of skill in the art, the amount of formamide in the prehybridization solution may be varied depending on the nature of the reaction obtained according to routine methods. Thus, a lower amount of formamide may result in more complete hybridization in terms of identifying hybridizing molecules than the same procedure using a larger amount of formamide. On the other hand, a strong hybridization band may be more easily visually identified by using more formamide.

A DNA probe generally between 50 and 500 bases in length should be isolated by electrophoresis in an agarose gel, the fragment excised from the gel, and recovered from the excised agarose. For a more detailed procedure, see Sambrook, supra. This purified fragment of DNA is then labeled (using, for example, the Megaprime labeling system according to the instructions of the manufacturer) to incorporate P³² in the DNA. The labeled probe is denatured by heating to 95 C for 5 minutes and immediately added to the membrane and prehybridization solution. The hybridization reaction should proceed for an appropriate time and under appropriate conditions, for example, for 18 hours at 37 C with gentle shaking or rotating. The membrane is rinsed (for example, in 2x SSC/0.3% SDS) and then washed in an appropriate wash solution with gentle agitation. The stringency desired will be a reflection of the conditions under which the membrane (filter) is washed.

In one embodiment, the nucleic acid sequence will be the sequence of any one of SEQ ID NOs: 1-6 or 13-14 and the hybridization stringency conditions will be high. In

In other embodiments, a promoter according to the invention will be a subsequence having at least 80%, at least 85%, at least 90%, at least 95%, at least 96%, at least 97%, at least 98% and at least 99% sequence identity with any one of SEQ ID NOs: 2&3. In another embodiment, a promoter according to the invention will be a subsequence having at least 80%, at least 85%, at least 90%, at least 95%, at least 96%, at least 97%, at least 98% and at least 99% sequence identity with SEQ ID NO: 3. A subsequence of any one of SEQ ID NOs: 2 and 3 may minimally comprise the -35 and -10 regions of the parent promoter, as illustrated in Figures 2 and 3 and Table 1-1. In certain embodiments, a subsequence of any of SEQ ID NOs:2&3 comprise the -35 and -10 regions of the parent promoter and further comprises the upstream UP elements of the parent promoter, as illustrated in Figures 2 and 3.

The term "identity" in the context of two nucleic acid sequences or polypeptides refers to nucleotides or amino acid residues in the two sequences that are the same when aligned for maximum correspondence, as measured using one of the following "sequence comparison algorithms." Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

An example of an algorithm that is suitable for determining sequence similarity is the BLAST algorithm, which is described in Altschul, et al., J. Mol. Biol. 215:403-410 (1990). Software for performing BLAST analyses is publicly available through the National Center for Biotechnology Information available on the world wide web (www) ncbi.nlm.nih.gov. The BLAST algorithm performs a statistical analysis of the similarity between two sequences (see, e.g., Karlin & Altschul, Proc. Nat'l. Acad. Sci. USA 90:5873-5787 (1993)).

### B. Coding Sequences of Interest

The promoters encompassed by the invention are operably linked to a nucleic acid encoding a protein of interest (i.e., a coding sequence of interest). The polypeptide encoded by the coding sequence is an enzyme.

In some embodiments, the enzyme is a protease, cellulase, hemicellulase, xylanase, amylase, glucoamylase, cutinase, phytase, laccase, lipase, isomerase, esterase, mannanase, carbohydrase, hydrolase, oxidase, permease, pullulanase, reductase, epimerase, tautomerase, transferase, kinase, phosphatase, or the like originating from bacteria or fungi.

In some embodiments, the enzyme is a cellulase. Cellulases are enzymes that hydrolyze the beta-D-glucosidic linkages in celluloses. Cellulolytic enzymes have been traditionally divided into three major classes: endoglucanases, exoglucanases or cellobiohydrolases and beta-glucosidases (Knowles, J. et al., TIBTECH 5:255-261 (1987)). Numerous cellulases have been described in the scientific literature, examples of which include: from Trichoderma reesei: Shoemaker, S. et al., Bio/Technology, 1:691-696, 1983, which discloses CBHI; Teeri, T. et al., Gene, 51:43-52, 1987, which discloses CBHII; Penttila, M. et al., Gene, 45:253-263, 1986, which discloses EGI; Saloheimo, M. et al., Gene, 63:11-22, 1988, which discloses EGII; Okada, M. et al., Appl. Environ. Microbiol., 64:555-563, 1988, which discloses EGIII; Saloheimo, M. et al., Eur. J. Biochem., 249:584-591, 1997, which discloses EGIV; and Saloheimo, A. et al., Molecular Microbiology, 13:219-228, 1994, which discloses EGV Exo-cellobiohydrolases and endoglucanases from species other than Trichoderma have also been described e.g., Ooi et al., 1990, which discloses the cDNA sequence coding for endoglucanase F1-CMC produced by Aspergillus aculeatus; Kawaguchi T et al., 1996, which discloses the cloning and sequencing of the cDNA encoding beta-glucosidase 1 from Aspergillus aculeatus; Sakamoto et al., 1995, which discloses the cDNA sequence encoding the endoglucanase CMCase-1 from Aspergillus kawachii IFO 4308; and Saarilahti et al., 1990 which discloses an endoglucanase from Erwinia carotovara.

In a particular embodiment, the cellulase to be expressed by a promoter of the invention is a cellulase disclosed in U.S. Pat. No. 6,2,87,839 and U.S. Pat. No. 6,562,612. In certain embodiments, the cellulase to be expressed is a cellulase comprising an amino acid sequence of SEQ ID NO: 1 of U.S. Pat. No. 6,562,612, a fragment or a derivative thereof having cellulolytic activity and greater than 70% sequence identity to an active portion of SEQ ID NO: 1 of U.S. Pat. No. 6,562,612.

In other embodiments, the enzyme is a protease, such as a serine, metallo, thiol or acid protease. In some embodiments, the protease will be a serine protease (e.g., subtilisin). Serine proteases are described in Markland, et al. (1983) Honne-Seyler's Z Physiol. Chem 364:1537-1540; Drenth, J. et al. (1972) Eur. J. Biochem. 26:177-181; U.S. Pat. Nos. 4,760,025 (RE 34,606), 5,182,204 and 6,312,936 and EP 0 323,299). Proteases that may be used in the invention are also described in, for example, U.S. Patent Publication No. 2010/0152088 and International Publication No. WO 2010/056635. Means for measuring proteolytic activity are disclosed in K. M. Kalisz, "Microbial Proteinases" ADVANCES IN BIOCHEMICAL ENGINEERING AND BIOTECHNOLOGY, A. Fiecht Ed. 1988.

In another embodiment, the protease to be expressed by a promoter of the invention is a protease comprising an amino acid sequence of SEQ ID NOs: 10, 12, 19, or 21, a fragment or a derivative thereof having proteolytic activity and greater than 70% sequence identity to an active portion of SEQ ID NO: 10, 12, 19, or 21. The nucleic acid sequences that encode SEQ ID NOs: 10, 12, 19, or 21are SEQ ID NOs: 9, 11, 18, and 20, respectively.

In other embodiments, the enzyme is an amylase, such as an amylase derived from *Trichoderma* (such as *T. reesei*), a *Trichoderma* glucoamylase, an amylase derived from *Bacillus* (such as *B. subtilis*), or an amylase derived from *Geobacillus* (such as G. *stearothermophilus*). Bacterial and fungal amylases are described in, for example, U.S. Patent No. 8,058,033, U.S. Patent Publication No. 2010/0015686, U.S. Patent Publication No. 2009/0314286, UK application No. 1011513.7, and International Application No. PCT/IB2011/053018.

In other embodiments, the enzyme is a xylanase. In certain embodiments, the xylanase is derived from *Trichoderma* (such as *T. reesei*). Bacterial and fungal xylanases are described in, for example, International Publication No. WO 2001/027252 and U.S. Patent No. 7,718,411.

In other embodiments, the enzyme is a phytase. In certain embodiments, the phytase is derived from *Citrobacter* (such as *C.freundii*) or *E. coli.* In other embodiments, they phytase may be a *Buttiauxella* phytase such as a *Buttiauxella agrestis* phytase. Phytases are described in, for example, International Publication Nos. WO 2006/043178, WO 2006/038062, WO 2008/097619, WO 2009/129489, WO 2006/038128, WO 2008/092901, WO 2009/129489, and WO 2010/122532.

The coding sequence may be either native or heterologous to a host cell. In addition, the coding sequence may encode a full-length protein, or a truncated form of a full-length protein. The invention is not limited to a particular coding sequence but encompasses numerous coding sequences, which are operably linked to a promoter of the invention.

### C. Signal Sequences

In some embodiments, especially when the coding sequence of interest codes for an extracellular enzyme, such as a cellulase, protease or starch degrading enzyme, a signal sequence may be linked to the N-terminal portion of the coding sequence. The signal may be used to facilitate the secretion of a DNA sequence. The signal sequence may be endogenous or exogenous to the host organism. The signal sequence may be one normally associated with the encoded polypeptide. In some embodiments, the signal sequence may be altered or modified as described in International Patent Publication Nos. WO 2011/014278 and WO 2010/123754. In some embodiments, the signal sequence comprises a signal sequence from a Streptomyces cellulase gene. In one embodiment, a preferred signal sequence is a S. lividans cellulase, celA (Bently et al., (2002) Nature 417:141-147). However, one skilled in the art is aware of numerous signal peptides which may be used depending on a protein to be expressed and secreted in a host organism.

### D. DNA Constructs and Vectors

The nucleic acid construct of the invention comprising a coding region of interest may be prepared synthetically by established standard methods, e.g., the phosphoramidite method described by Beaucage and Caruthers, (1981) Tetrahedron Letters 22:1859-1869, or the method described by Matthes et al., (1984) EMBO Journal 3: 801-805. The nucleic acid construct may be of mixed synthetic and genomic origin and may be prepared by ligating fragments of synthetic or genomic DNA. The nucleic acid construct may also be prepared by polymerase chain reaction using specific primers, for instance as described in U.S. Pat. No. 4,683,202 or Saiki et al., Science 239 (1988), 487-491.

A DNA construct of the invention may be inserted into a vector, such as an expression vector. A variety of vectors suitable for the cloning, transformation and expression of polypeptides in fungus, yeast and bacteria are known by those of skill in the art. Typically, the vector or cassette will comprise a promoter of the invention, optionally a signal sequence, a coding region of interest and a terminator sequence. In preferred embodiments, the vector will include one or more cloning sties located between the signal sequence and the terminator sequences.

### E. Transformation

A vector of the invention will be transformed into a host cell. General transformation techniques are known in the art (Ausubel et al., 1994, CURRENT PROTOCOLS IN MOLECULAR BIOLOGY and Campbell et al., 1989 Curr. Genet 16:53-56). Some of these general techniques include, but are not limited to the use of a particle or gene gun (biolistics), permeabilization of filamentous fungi cells walls prior to the transformation process (e.g., by use of high concentrations of alkali, e.g., 0.05 M to 0.4 M CaCl₂ or lithium acetate), protoplast fusion, electroporation, or agrobacterium mediated transformation (U.S. Pat. No. 6,255,115) and the treatment of protoplasts or spheroplasts with polyethylene glycol and CaCl.sub.2 is described in Campbell, et al., (1989) Curr. Genet. 16:53-56, 1989 and Penttila, M. et al., (1988) Gene, 63:11-22.

Transformation and expression methods for bacteria are disclosed in Brigidi, DeRossi, Bertarini, Riccardi and Matteuzzi, (1990), FEMS Microbiol. Lett. 55: 135-138. A preferred general transformation and expression protocol for protease deleted Bacillus strains is provided in Ferrari et al., U.S. Pat. No. 5,264,366.

Transformation and expression in Streptomyces can be found in Hopwood et al., GENETIC MANIPULATION OF STREPTOMYCES: A LABORATORY MANUAL, (1985) John Innis Foundation, Norwich UK.

In other embodiments, transformation and expression in Aspergillus and Trichoderma is described in, for example U.S. Pat. No. 5,364,770; U.S. Pat. No. 6,022,725; and Nevalainen et al., 1992, The Molecular Biology of Trichoderma and its Application to the Expression of Both Homologous and Heterologous Genes, in MOLECULAR INDUSTRIAL MYCOLOGY, Eds. Leon and Berka, Marcel Dekker, Inc. pp. 129-148.

### F. Host cells

Host cells that may be used according to the invention include both bacterial and fungal cells. Preferred fungal host cells include filamentous fungal cells such as Aspergillus and Trichoderma cells. Preferred bacterial host cells include both gram positive and gram negative cells, including Bacillus, Mycobacterium, Actinomyces and Streptomyces cells. Host cells also include, without limitation, E. coli, Pseudomonas spp. (e.g., P. aeruginoa and P. alcaligenes), Streptomyces spp., (e.g., Streptomyces lividans), B. subtilis, B. licheniformis, B. lentus, B. brevis, B. stearothermophilus, B. alkalophilus, B. amyloliquefaciens, B. coagulans, B. circulans, B. lautus, B. megatherium, and B. thuringiensis.

### G. Cell Culture

Host cells and transformed cells can be cultured in conventional nutrient media. The culture media for transformed host cells may be modified as appropriate for activating promoters and selecting transformants. The specific culture conditions, such as temperature, pH and the like, may be those that are used for the host cell selected for expression, and will be apparent to those skilled in the art. In addition, preferred culture conditions may be found in the scientific literature such as Sambrook, (1982) supra; Kieser, T, M J. Bibb, M J. Buttner, K F Chater, and D. A. Hopwood (2000) PRACTICAL STREPTOMYCES GENETICS. John Innes Foundation, Norwich UK; Harwood, et al., (1990) MOLECULAR BIOLOGICAL METHODS FOR BACILLUS, John Wiley and/or from the American Type Culture Collection (ATCC; "http://www.atcc.org/"). Stable transformants of fungal host cells, such as Trichoderma cells can generally be distinguished from unstable transformants by their faster growth rate or the formation of circular colonies with a smooth rather than ragged outline on solid culture medium.

### H. Recovery of Expressed Polypeptides

A polypeptide produced by the transformed host cell may be recovered from the culture medium by conventional procedures including separating the host cells from the medium by centrifugation or filtration, or if necessary, disrupting the cells and removing the supernatant from the cellular fraction and debris. Typically after clarification, the proteinaceous components of the supernatant or filtrate are precipitated by means of a salt, e.g., ammonium sulphate. The precipitated proteins are then solubilized and may be purified by a variety of chromatographic procedures, e.g., ion exchange chromatography, gel filtration chromatography, affinity chromatography, and other art-recognized procedures.

### I. Construct Assembly

In one general embodiment, the present invention involves assembling a DNA construct in vitro, followed by direct cloning of such construct into competent host cells (e.g., Bacillus host cells) such that the construct becomes integrated into the host genome. For example, in some embodiments PCR fusion and/or ligation are employed to assemble a DNA construct in vitro. In a preferred embodiment, the DNA construct is a non-plasmid DNA construct. In another embodiment, the DNA construct comprises a DNA into which a mutation has been introduced. This construct is then used to transform host cells. In this regard, highly competent mutants of a host cell (e.g., Bacillus) are preferably employed to facilitate the direct cloning of the constructs into the cells. For example, Bacillus carrying the comK gene under the control of a xylose-inducible promoter (Pxyl-comK) can be reliably transformed with very high efficiency, as described herein. Any suitable method known in the art may be used to transform the cells. The DNA construct may be inserted into a vector (i.e., a plasmid), prior to transformation. In some preferred embodiments, the circular plasmid is cut using an appropriate restriction enzyme (i.e., one that does not disrupt the DNA construct). Thus, in some embodiments, circular plasmids find use with the present invention. However, in alternative embodiments, linear plasmids are used. In some embodiments, the DNA construct (i.e., the PCR product) is used without the presence of plasmid DNA.

In order to further illustrate the present invention and advantages thereof, the following specific examples are given with the understanding that they are being offered to illustrate the present invention and should not be construed in any way as limiting its scope.

### EXAMPLES

### Example 1: Generation of Bacillus subtilis strains expressing proteins from ribosomal RNA and protein promoters

The coding sequences of Green fluorescence Protein (GPF), and two subtilisin proteases, FNA (*B. amyloliquefaciens* subtilisin BPN'-Y217L) and ER11 (described in WO2010/056635A1), were fused to *Bacillus subtilis* ribosomal RNA or protein promoters to test protein expression in *Bacillus subtilis* strains BG8000 (*ΔnprE, degU(Hy)32, ΔaprE, spoIIE312 amyE::PxylRA-comK-eryR)* and BG8010 (*ΔnprE, degU(Hy)32, ΔaprE, spoIIE312 amyE::PxylRA-comK-eryR oppA: phleoR).* The expression of the proteins from the ribosomal RNA and protein promoters was compared to that obtained from expression with subtilisin promoter aprE (Transcription of Bacillus subtilis subtilisin and expression of subtilisin in sporulation mutants. E Ferrari, D J Henner, M Perego, and J A Hoch, J Bacteriol. 1988 January; 170(1): 289-295).

The promoters shown in Table 1-1 were amplified by PCR from the *Bacillus subtilis* 168 chromosomal DNA and transcriptionally fused to the genes for the target molecules (ER11, FNA or GFP). BG8010 or BG8000 strains were transformed with the cassette comprising promoter, gene of interest and antibiotic marker and transformants were selected on LB agar plates containing 5µg/ml chloramphenicol. BG8010 or BG8000 strains were also transformed with constructs comprising *aprE* promoter fused to the target molecule genes and transformants were selected on LB agar plates containing 5µg/ml chloramphenicol. The strains carrying the construct with the subtilisin promoter were amplified on LB agar plates containing 25 µg/ml chloramphenicol to increase the number of copies of the cassette, while the strains carrying the ribosomal promoters were reisolated on plates containing 5µg/ml chloramphenicol.

| **Table 1-1: List of promoters (the -35 and -10 consensus sequences are bold and underlined)** | | |
|---|---|---|
| Ribosomal RNA Promoters | | |
| **Name** | **Sequence** | **SEQ ID NO** |
| P1 *rrnB* | ATAGATTTTTTTAAAAAACTATTGC AATAAATAAATACAGGTGTTATATTA TTAAACG TCGCTG | 1 |
| P1 *rrnI* | CACATACAGCCTAAATTGGGTGTTGA CCTTTTGATAATATCCGTGATATATT ATTATTCG TCGCTG | 2 |
| P2 *rrnI* | TTAAATACTTTGAAAAAAGTTGTTGA CTTAAAAGAAGCTAAATGTTATAGTA ATAAAG CTGCTT | 3 |
| P1 *rrnE* | ATAAAAAAATACAGGAAAAGTGTTG ACCAAATAAAACAGGCATGGTATATT ATTAAACG TCGCTG | 4 |
| P2 *rrnE* | AACAAAAAAGTTTTCCTAAGGTGTTT ACAAGATTTTAAAAATGTGTATAATA AGAAAAG TCGAAT | 5 |
| P3 *rrnE* | TCGAAAAAACATTAAAAAACTTCTTG ACTCAACATCAAATGATAGTATGATA GTTAAG TCGCTC | 6 |

The nucleotide and amino acid sequences of the target molecules are shown below:

### Nucleotide sequence of the GFP gene fused to the ribosomal RNA and protein promoters

ACAGAATAGTCTTTTAAGTAAGTCTACTCTGAATTTTTTAAAAGGAGAGGGTAA
AGAGTGAATAGAAATGTTCTTAAAAATACTGGTCTGAAGGAGATCATGTCAGCG
AAAGCGTCTGTGGAAGGTATTGTGAACAATCACGTATTCTCAATGGAGGGTTTG
GAAAGGAAATGTTTTGTTTGGTAACCAGTTAATGCAAATTCGAGTTACCAAAGG
CGGCCCACTTCCATTTGCCTTCGACATCGTAAGCATCGCCTTCCAGTACGGCAAT
CGCACCTTTACGAAATATCCTGATGATATCGCCGACTATTTCGTGCAATCGTTTCC
AGCGGGCTTTTTCTATGAAAGAAATCTGCGGTTTGAAGATGGCGCAATCGTTGAT
ATACGTTCAGACATCAGTCTGGAGGATGACAAGTTTCACTATAAAGTGGAGTATC
GAGGAAACGGATTTCCGTCTAACGGGCCTGTCATGCAAAAAGCTATTTTGGGCAT
GGAGCCGTCTTTTGAAGTGGTTTATATGAATAGCGGCGTCCTTGTAGGGGAAGTG
GATTTAGTTTATAAGCTGGAAAGCGGAAATTATTATTCATGCCATATGAAAACCT
TCTATAGATCAAAGGGCGGAGTGAAAGAATTTCCAGAATATCACTTTATTCATCA

TAGACTGGAGAAAACGTATGTTGAAGAAGGTTCTTTCGTCGAACAGCATGAGAC
AGCGATCGCTCAGCTTACCACAATAGGCAAACCGCTGGGTTCGCTCCATGAATGG
GTTTAA (SEQ ID NO:7)

### Amino acid sequence of the GFP expressed from the ribosomal RNA and protein promoters

VNRNVLKNTGLKEIMSAKASVEGIVNNHVFSMEGFGKGNVLFGNQLMQIRVTKGGP
LPFAFDIVSIAFQYGNRTFTKYPDDIADYFVQSFPAGFFYERNLRFEDGAIVDIRSDISL
EDDKFHYKVEYRGNGFPSNGPVMQKAILGMEPSFEVVYMNSGVLVGEVDLVYKLE
SGNYYSCHMKTFYRSKGGVKEFPEYHFIHHRLEKTYVEEGSFVEQHETAIAQLTTIG
KPLGSLHEWV (SEQ ID NO:8)

### Nucleotide sequence of the FNA subtilisin protease gene fused to the ribosomal RNA and protein promoters

ACAGAATAGTCTTTTAAGTAAGTCTACTCTGAATTTTTTTAAAAGGAGAGGGTAA
AGAGTGAGAAGCAAAAAATTGTGGATCAGTTTGCTGTTTGCTTTAGCGTTAATCT
TTACGATGGCGTTCGGCAGCACATCCTCTGCCCAGGCGGCAGGGAAATCAAACG
GGGAAAAGAAATATATTGTCGGGTTTAAACAGACAATGAGCACGATGAGCGCCG
CTAAGAAGAAAGATGTCATTTCTGAAAAAGGCGGGAAAGTGCAAAAGCAATTCA
AATATGTAGACGCAGCTTCAGCTACATTAAACGAAAAAGCTGTAAAAGAATTGA
AAAAAGACCCGAGCGTCGCTTACGTTGAAGAAGATCACGTAGCACATGCGTACG
CGCAGTCCGTGCCTTACGGCGTATCACAAATTAAAGCCCCTGCTCTGCACTCTCA
AGGCTACACTGGATCAAATGTTAAAGTAGCGGTTATCGACAGCGGTATCGATTCT
TCTCATCCTGATTTAAAGGTAGCAGGCGGAGCCAGCATGGTTCCTTCTGAAACAA
ATCCTTTCCAAGACAACAACTCTCACGGAACTCACGTTGCCGGCACAGTTGCGGC
TCTTAATAACTCAATCGGTGTATTAGGCGTTGCGCCAAGCGCATCACTTTACGCT
GTAAAAGTTCTCGGTGCTGACGGTTCCGGCCAATACAGCTGGATCATTAACGGAA
TCGAGTGGGCGATCGCAAACAATATGGACGTTATTAACATGAGCCTCGGCGGAC
CTTCTGGTTCTGCTGCTTTAAAAGCGGCAGTTGATAAAGCCGTTGCATCCGGCGT
CGTAGTCGTTGCGGCAGCCGGTAACGAAGGCACTTCCGGCAGCTCAAGCACAGT
GGGCTACCCTGGTAAATACCCTTCTGTCATTGCAGTAGGCGCTGTTGACAGCAGC
AACCAAAGAGCATCTTTCTCAAGCGTAGGACCTGAGCTTGATGTCATGGCACCTG
GCGTATCTATCCAAAGCACGCTTCCTGGAAACAAATACGGCGCGTTGAACGGTA
CATCAATGGCATCTCCGCACGTTGCCGGAGCGGCTGCTTTGATTCTTTCTAAGCA
CCCGAACTGGACAAACACTCAAGTCCGCAGCAGTTTAGAAAACACCACTACAAA

ACTTGGTGATTCTTTCTACTATGGAAAAGGGCTGATCAACGTACAGGCGGCAGCT
CAGTAA (SEQ ID NO:9)

### Amino acid sequence of the FNA subtilisin protease expressed from the ribosomal RNA and protein promoters

VRSKKLWISLLFALALIFTMAFGSTSSAQAAGKSNGEKKYIVGFKQTMSTMSAAKKK
DVISEKGGKVQKQFKYVDAASATLNEKAVKELKKDPSVAYVEEDHVAHAYAQSVP
YGVSQIKAPALHSQGYTGSNVKVAVIDSGIDSSHPDLKVAGGASMVPSETNPFQDNN
SHGTHVAGTVAALNNSIGVLGVAPSASLYAVKVLGADGSGQYSWIINGIEWAIANN
MDVINMSLGGPSGSAALKAAVDKAVASGVVVVAAAGNEGTSGSSSTVGYPGKYPS
VIAVGAVDSSNQRASFSSVGPELDVMAPGVSIQSTLPGNKYGALNGTSMASPHVAG
AAALILSKHPNWTNTQVRSSLENTTTKLGDSFYYGKGLINVQAAAQ (SEQ ID NO:10)

### Nucleotide sequence of the ER11 subtilisin protease gene fused to the ribosomal RNA and protein promoters

GAATAGTCTTTTAAGTAAGTCTACTCTGAATTTTTTTAAAAGGAGAGGGTAAAGA
GTGAGAAGCAAAAAATTGTGGATCAGCTTGTTGTTTGCGTTAACGTTAATCTTTA
CGATGGCGTTCAGCAACATGTCTGCGCAGGCTGCTGAAGAAGCAAAAGAAAAT
ATTTAATTGGCTTTAATGAGCAGGAAGCTGTCAGTGAGTTTGTAGAACAAGTAGA
GGCAAATGACGGCGTCGCCATTCTCTCTGAGGAAGAGGAAGTCGAAATTGAATT
GCTTCATGAATTTGAAACGATTCCTGTTTTATCCGTTGAGTTAAGCCCAGAAGAT
GTGGACGCGCTTGAACTCGATCCAGCGATTTCTTATATTGAAGAGGATGCAGAAG
TAACGACAATGGCGCAATCGGTACCATGGGGAATTAGCCGTGTGCAAGCCCCAG
CTGCCCATAACCGTGGATTGACAGGTTCTGGTGTAAAAGTTGCTGTCCTCGATAC
AGGTATTTCCACTCATCCAGACTTAAATATTCGTGGTGGCGCTAGCTTTGTACCA
GGGGAACCATCCACTCAAGATGGGAATGGGCATGGCACGCATGTGGCTGGGACG
ATTGCTGCTTTAAACAATTCGATTGGCGTTCTTGGCGTAGCACCGAACGCGGAAC
TATACGCTGTTAAAGTATTAGGGGCGAGCGGTATGGGTTCGGTCAGCTCGATTGC
CCAAGGATTGGAATGGGCAGGGAACAATGTTATGCACGTTGCTAATTTGAGTTTA
GGACTGCAGGCACCAAGTGCCACACTTGAGCAAGCTGTTAATAGCGCGACTTCT
AGAGGCGTTCTTGTTGTAGCGGCATCTGGCAATTCAGGTGCAGGCTCAATCAGCT
ATCCGGCCCGTTATGCGAACGCAATGGCAGTCGGAGCTACTGACCAAAACAACA
ACCGCGCCAGCTTTTCACAGTATGGCGCAGGGCTTGACATTGTCGCACCAGGTGT
AAACGTGCAGAGCACATACCCAGGTTCAACGTATGCCAGCTTAAACGGTACATC
GATGGCTACTCCTCATGTTGCAGGTGCAGCAGCCCTTGTTAAACAAAAGAACCCA

TCTTGGTCCAATGTCCAAATCCGCAATCATCTAAAGAATACGGCAACGAGCTTAG
GAAGCACGAACTTGTATGGAAGCGGACTTGTCAATGCAGAAGCGGCAACACGTT
AA (SEQ ID NO:11)

### Amino acid sequence of the ER11 subtilisin protease expressed from the ribosomal RNA and protein promoters

VRSKKLWISLLFALTLIFTMAFSNMSAQAAEEAKEKYLIGFNEQEAVSEFVEQVEAN
DGVAILSEEEEVEIELLHEFETIPVLSVELSPEDVDALELDPAISYIEEDAEVTTMAQSV
PWGISRVQAPAAHNRGLTGSGVKVAVLDTGISTHPDLNIRGGASFVPGEPSTQDGNG
HGTHVAGTIAALNNSIGVLGVAPNAELYAVKVLGASGMGSVSSIAQGLEWAGNNV
MHVANLSLGLQAPSATLEQAVNSATSRGVLVVAASGNSGAGSISYPARYANAMAV
GATDQNNNRASFSQYGAGLDIVAPGVNVQSTYPGSTYASLNGTSMATPHVAGAAA
LVKQKNPSWSNVQIRNHLKNTATSLGSTNLYGSGLVNAEAATR (SEQ ID NO:12)

The different strains constructed from the fusion of the promoters to the target genes are listed in Table 1-2 below. 1/2/3 indicates that the three promoters were cloned in tandem to drive the expression of the target molecule. The mark "+ G" indicates the use of the nucleotide guanine as transcription start site instead of adenine.

| **Table 1-2: List of strains constructed** |
|---|
| BG8000 P*aprE*-FNA |
| BG8000 P*aprE*-FNA |
| BG8000 P*rrnI* 2-FNA |
| BG8000 P*rrnI* 2-FNA |
| BG8010 P*aprE*-FNA |
| BG8010 P*aprE*-FNA |
| BG8010 P*rrnI* 2-FNA |
| BG8010 P*rrnI 2*-FNA |
| BG8000 P*rrnI 2*-ER11 |
| BG8000 P*rrnI 2*-ER11 |
| BG8010 P*rrnI 2*-ER11 |
| BG8010 P*rrnI 2*-ER11 |
| BG8000 P*aprE*-ER11 |
| BG8000 P*aprE*-ER11 |
| BG8010 P*aprE*-ER11 |
| BG8010 P*aprE*-ER11 |
| BG8010 P*rrnI 1*/*2*-FNA |

| **Table 1-2: List of strains constructed** |
|---|
| BG8010 P*rrnI 2*-FNA |
| BG8010 *PrrnI 1*/*2*+*G*-FNA |
| BG8010 P*rrnE 2*-FNA |
| BG8010 P*rrnB1*-FNA |
| BG8010 P*rrnE 1*/*2*/*3*-FNA |
| BG8010 P*rrnI* 1/2-GFP |
| BG8010 P*rrnI* 2-GFP |
| BG8010 P*rrnI* 1/2+G-GFP |
| BG8010 P*rrnE 1*/*2*/*3*-GFP |
| BG8010 P*rrnE 2*/*3*-GFP |
| BG8010 P*rrnB1*-GFP |
| BG8010 P*aprE*-GFP |

### Example 2: Cell density measurements of GFP, FNA and ER11 expressing strains

To test for cell growth, one colony each of the constructed strains was inoculated in Luria Broth containing 5µg/ml chloramphenicol (for strains expressing from ribosomal RNA promoters) or 25 µg/ml chloramphenicol (for strains expressing from *aprE* promoters) and grown overnight at 30°C. One ml of each pre-culture was used to inoculate 32 ml of 2xSNB medium (see composition below) and grown at 37°C in shake flasks at 280 rpm, 70% humidity. At hourly intervals from 4 hours to 8 hours of growth, optical densities of each culture was measured at 600 nm using a SpectraMax reader. The cell density measurements of GFP, FNA, and ER11 expressing strains are shown in Figures 4 (GFP), 5 and 6 (FNA), and 7A and 7B (ER11). The growth of strains containing the different constructs was comparable.
2xSNB medium:
Stock solutions (filter sterilized): 25x SNB salts-CaCl₂*2H₂O (3.7 g/L), FeSO₄*7H₂O (9.6 mg/L), MnCl₂*4H₂O (6 mg/L), KCl (25 g/L), MgSO₄*7H₂O (3.26 g/L), Maltrin 150 10% Prepare 500mL of 16 g/L solution of Difco Nutrient Broth, autoclave, add 20mL 25xSNB salts, and 25mL 10% Maltrin 150.

### Example 3: Protein expression of GFP, FNA, and ER11 from ribosomal promoters

The extracellular production of ER11, FNA or intracellular expression of GFP driven by the selected promoters was tested in BG8000 and BG8010 strains. The cells were grown as described for the cell density measurements in Example 2. At hourly intervals from 4 hours to 8 hours of growth, supernatants of cultures were analyzed for AAPF activity (subtilisin expression). GFP expression was measured as Relative Fluorescence Units (RFU) expressed in the cell.

The AAPF activity of a sample was measured as the rate of hydrolysis of N-succinyl-L-alanyl-L-alanyl-L-prolyl-L-phenyl-p-nitroanilide (suc-AAPF-pNA). The reagent solutions used were: 100 mM Tris/HCl, pH 8.6, containing 0.005% TWEEN®-80 (Tris dilution buffer and 160 mM suc-AAPF-pNA in DMSO (suc-AAPF-pNA stock solution) (Sigma: S-7388). To prepare a suc-AAPF-pNA working solution, 1 ml suc-AAPF-pNA stock solution was added to 100 ml Tris/ HCl buffer and mixed well for at least 10 seconds. The assay was performed by diluting the samples in the assay buffer (5 µl in 195 µl). Then, 180µl of assay buffer with AAPF substrate was added to 20 µl of the diluted sample arrayed in a microtiter plate. The solutions were mixed for 5 sec., and the absorbance change in kinetic mode (20 readings in 5 minutes) was read at 405 nm in a SpectraMax reader, at 25°C.

For measuring GFP expression in RFU, 150 µl of each culture sample was loaded into a microtiter plate and fluorescence measurements were taken using the SpectraMax reader using an excitation wavelength at 485 nm, emission wavelength at 508 nm, with a cutoff at 495 nm.

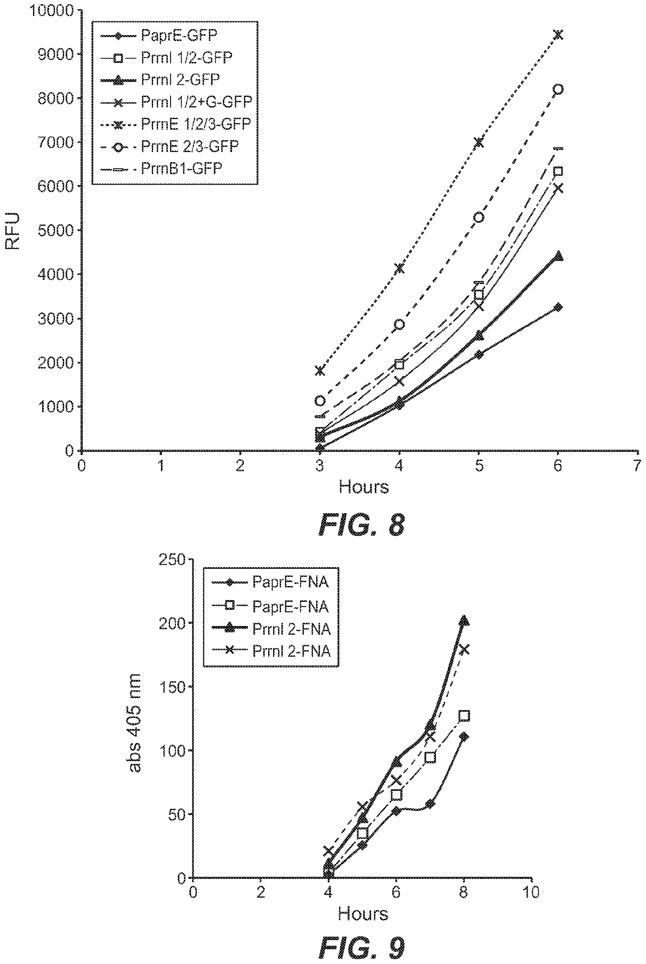

Expression of GFP, FNA, and ER11 from the different promoters is shown in Figures 8 (GFP), 9 and 10 (FNA), and 11A and 11B (ER11). Protein expression from non-amplified ribosomal RNA promoter and protein promoter was higher than that seen from amplified *aprE* promoter.

### Example 4: Protein expression from SigmaA dependent promoter

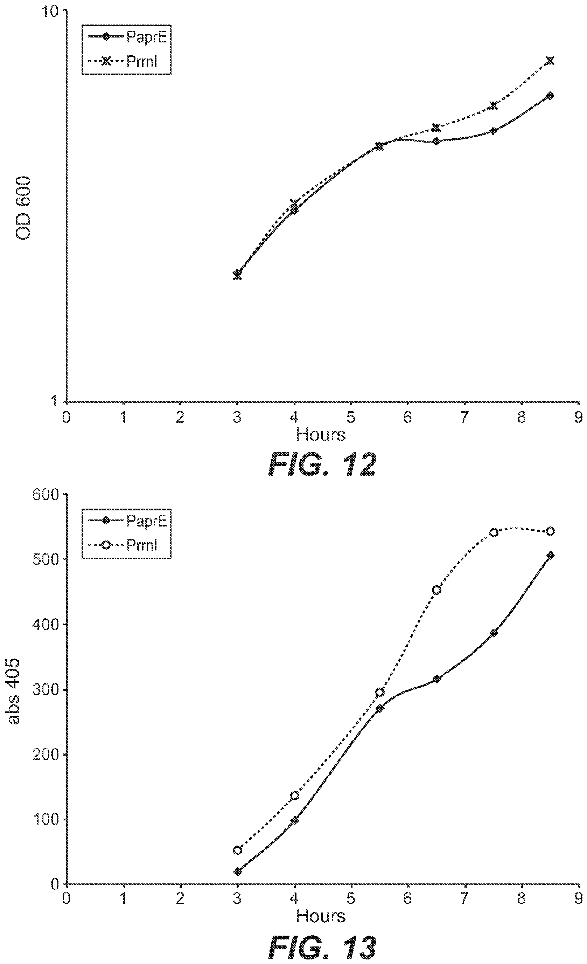

As different levels of protein expression are observed from different promoters, this experiment compared FNA expression amplified *apr*E promoter and unamplified *rrnI* P2 promoter. BG8010 strains expressing FNA from *apr*E were amplified using 25µg/mL chloramphenicol, while strains expressing FNA from *rrnI* P2 were reisolated on 5µg/mL chloramphenicol as described in Example 1. Cell density measurements and FNA expression was studied as described in Examples 2 and 3 respectively. Results are shown in Figures 12 and 13. Cell growth from all strains was comparable, but FNA expression from unamplified *rrnI* P2 promoter was higher that from amplified *apr*E promoters.

### Example 5: FNA expression from BG8010 strains containing single copy integrant

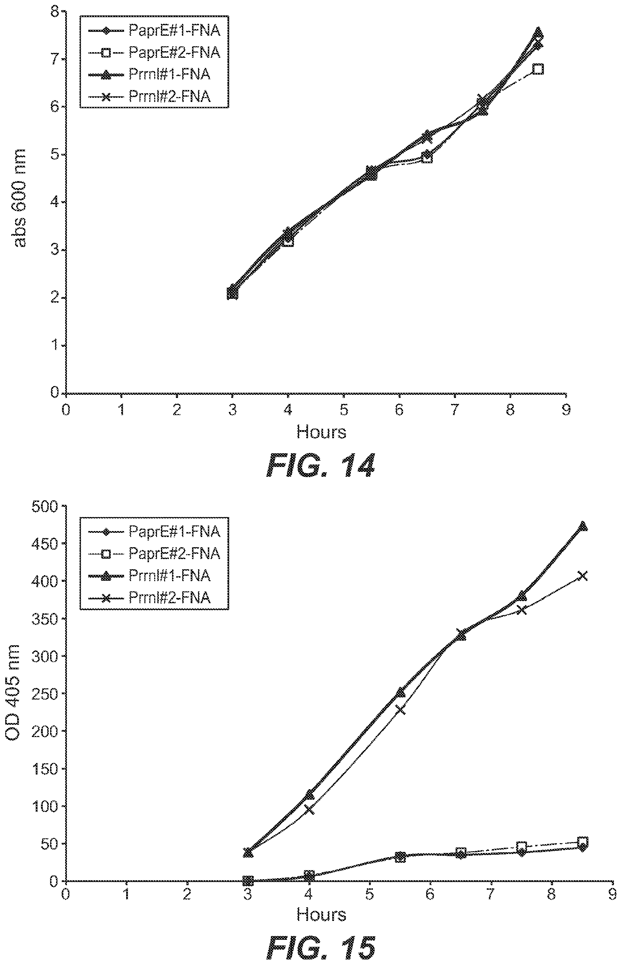

To test whether *rrnI* P2 promoter could be used for protein expression without the use of antibiotic marker, a single copy integrant containing either P*rrnI* P2-FNA SpcR or P*aprE-*FNA CatR cassette was integrated in the BG8010 strain by double cross over integration. The antibiotic marker genes flanked by lox sequences were subsequently removed using cre-lox recombinase. Transformants of constructed strains were grown as described in Example 2 and cell density measurements and FNA expression were studied as described in Example 2 and 3 respectively. Results shown in Figure 14 indicate that growth of strains containing either the *rrnI* P2 or *apr*E promoter was comparable, but FNA expression from P*rrnI*-P2 was higher than from *apr*E (Figure 15). These studies demonstrate that P*rrnI*-P2 is a strong promoter that can deliver high amount of mRNA of the target molecule. The advantage of using this promoter consists in delivering high amount of transcript without the need of the amplification of the construct and without the use of the antibiotic marker.

**Table 2-1: Promoter sequences are shown for rpsD, rpsJ, and rpoD (P1). -35 and -10 sequences are shown in bold and underlined for each promoter. For rpsJ, two promoters are available (PI and P2). The -35 and -10 sequences for rpsJ P1 are upstream (i.e., 5') of the -35 and -10 sequences for rpsJ P2 sequences.**

| **Table 2-1: List of promoters (the -35 and -10 consensus sequences are bold and underlined)** | | |
|---|---|---|
| **Name** | **Sequence** | **SEQ ID NO** |
| Ribosomal protein promoters | | |
| *rpsD* | GTTTTTATCACCTAAAAGTTTACCACT AATTTTTGTTTATTATATCATAAACGG TGAAGCAATAATGGAGGAATGGTTGA CTTCAAAACAAATAAATTATATAATG ACCTTT | 13 |
| *rpsJ* | GTACCGTGTGTTTTCATTTCAGGGAAA CATGACTTAATTGTTCCTGCAGAAATA TCGAAACAGTATTATCAAGAACTTGA GGCACCTGAAAAGCGCTGGTTTCAAT TTGAGAATTCAGCTCACACCCCGCAT ATTGAGGAGCCATCATTATTCGCGAA CACATTAAGTCGGCATGCACGCAACC ATTTATGATAGATCCTTGATAAATAA GAAAAACCCCTGTATAATAAAAAAA GTGTGCAAATGATGCATATTTTAAAT AAGTCTTGCAACATGCGCCTATTTTCT GTATAATGGTGTATA | 14 |

| Sigma factor promoter | | |
|---|---|---|
| *rpoD* (P1) | AACATATAACTCAGGACGCTCTATCC TGGGTTTTTGGCTGTGCCAAAAGGGA ATAATGAAAAACAATAGCATCTTTGT GAAGTTTGTATTATAATAAAAAATT | 15 |

### Nucleotide sequence of the GFP gene fused to the ribosomal protein promoters

ACAGAATAGTCTTTTAAGTAAGTCTACTCTGAATTTTTTAAAAGGAGAGGGTAA
AGAGTGAATAGAAATGTTCTTAAAAATACTGGTCTGAAGGAGATCATGTCAGCG
AAAGCGTCTGTGGAAGGTATTGTGAACAATCACGTATTCTCAATGGAGGGTTTG
GAAAGGAAATGTTTTGTTTGGTAACCAGTTAATGCAAATTCGAGTTACCAAAGG
CGGCCCACTTCCATTTGCCTTCGACATCGTAAGCATCGCCTTCCAGTACGGCAAT
CGCACCTTTACGAAATATCCTGATGATATCGCCGACTATTTCGTGCAATCGTTTCC
AGCGGGCTTTTTCTATGAAAGAAATCTGCGGTTTGAAGATGGCGCAATCGTTGAT
ATACGTTCAGACATCAGTCTGGAGGATGACAAGTTTCACTATAAAGTGGAGTATC
GAGGAAACGGATTTCCGTCTAACGGGCCTGTCATGCAAAAAGCTATTTTGGGCAT

GGAGCCGTCTTTTGAAGTGGTTTATATGAATAGCGGCGTCCTTGTAGGGGAAGTG
GATTTAGTTTATAAGCTGGAAAGCGGAAATTATTATTCATGCCATATGAAAACCT
TCTATAGATCAAAGGGCGGAGTGAAAGAATTTCCAGAATATCACTTTATTCATCA
TAGACTGGAGAAAACGTATGTTGAAGAAGGTTCTTTCGTCGAACAGCATGAGAC
AGCGATCGCTCAGCTTACCACAATAGGCAAACCGCTGGGTTCGCTCCATGAATGG
GTTTAA (SEQ ID NO:16)

### Amino acid sequence of the GFP expressed from the ribosomal protein promoters

VNRNVLKNTGLKEIMSAKASVEGIVNNHVFSMEGFGKGNVLFGNQLMQIRVTKGGP
LPFAFDIVSIAFQYGNRTFTKYPDDIADYFVQSFPAGFFYERNLRFEDGAIVDIRSDISL
EDDKFHYKVEYRGNGFPSNGPVMQKAILGMEPSFEVVYMNSGVLVGEVDLVYKLE
SGNYYSCHMKTFYRSKGGVKEFPEYHFIHHRLEKTYVEEGSFVEQHETAIAQLTTIG
KPLGSLHEWV (SEQ ID NO:17)

### Nucleotide sequence of the FNA subtilisin protease gene fused to the ribosomal protein promoters

ACAGAATAGTCTTTAAGTAAGTCTACTCTGAATTTTTTAAAAGGAGAGGGTAA
AGAGTGAGAAGCAAAAATTGTGGATCAGTTTGCTGTTTGCTTTAGCGTTAATCT
TTACGATGGCGTTCGGCAGCACATCCTCTGCCCAGGCGGCAGGGAAATCAAACG
GGGAAAAGAAATATATTGTCGGGTTTAAACAGACAATGAGCACGATGAGCGCCG
CTAAGAAGAAAGATGTCATTTCTGAAAAGGCGGGAAAGTGCAAAAGCAATTCA
AATATGTAGACGCAGCTTCAGCTACATTAAACGAAAAAGCTGTAAAAGAATTGA
AAAAAGACCCGAGCGTCGCTTACGTTGAAGAAGATCACGTAGCACATGCGTACG
CGCAGTCCGTGCCTTACGGCGTATCACAAATTAAAGCCCCTGCTCTGCACTCTCA
AGGCTACACTGGATCAAATGTTAAAGTAGCGGTTATCGACAGCGGTATCGATTCT
TCTCATCCTGATTTAAAGGTAGCAGGCGGAGCCAGCATGGTTCCTTCTGAAACAA
ATCCTTTCCAAGACAACAACTCTCACGGAACTCACGTTGCCGGCACAGTTGCGGC
TCTTAATAACTCAATCGGTGTATTAGGCGTTGCGCCAAGCGCATCACTTTACGCT
GTAAAAGTTCTCGGTGCTGACGGTTCCGGCCAATACAGCTGGATCATTAACGGAA
TCGAGTGGGCGATCGCAAACAATATGGACGTTATTAACATGAGCCTCGGCGGAC
CTTCTGGTTCTGCTGCTTTAAAAGCGGCAGTTGATAAAGCCGTTGCATCCGGCGT
CGTAGTCGTTGCGGCAGCCGGTAACGAAGGCACTTCCGGCAGCTCAAGCACAGT
GGGCTACCCTGGTAAATACCCTTCTGTCATTGCAGTAGGCGCTGTTGACAGCAGC
AACCAAAGAGCATCTTTCTCAAGCGTAGGACCTGAGCTTGATGTCATGGCACCTG
GCGTATCTATCCAAAGCACGCTTCCTGGAAACAAATACGGCGCGTTGAACGGTA

CATCAATGGCATCTCCGCACGTTGCCGGAGCGGCTGCTTTGATTCTTTCTAAGCA
CCCGAACTGGACAAACACTCAAGTCCGCAGCAGTTTAGAAAACACCACTACAAA
ACTTGGTGATTCTTTCTACTATGGAAAAGGGCTGATCAACGTACAGGCGGCAGCT
CAGTAA (SEQ ID NO:18)

### Amino acid sequence of the FNA subtilisin protease expressed from the ribosomal protein promoters

VRSKKLWISLLFALALIFTMAFGSTSSAQAAGKSNGEKKYIVGFKQTMSTMSAAKKK
DVISEKGGKVQKQFKYVDAASATLNEKAVKELKKDPSVAYVEEDHVAHAYAQSVP
YGVSQIKAPALHSQGYTGSNVKVAVIDSGIDSSHPDLKVAGGASMVPSETNPFQDNN
SHGTHVAGTVAALNNSIGVLGVAPSASLYAVKVLGADGSGQYSWIINGIEWAIANN
MDVINMSLGGPSGSAALKAAVDKAVASGVVVVAAAGNEGTSGSSSTVGYPGKYPS
VIAVGAVDSSNQRASFSSVGPELDVMAPGVSIQSTLPGNKYGALNGTSMASPHVAG
AAALILSKHPNWTNTQVRSSLENTTTKLGDSFYYGKGLINVQAAAQ (SEQ ID NO:19)

### Nucleotide sequence of the ER11 subtilisin protease gene fused to the ribosomal protein promoters

GAATAGTCTTTAAGTAAGTCTACTCTGAATTTTTTAAAGGAGAGGTAAAGA
GTGAGAAGCAAAAATTGTGGATCAGCTTGTTGTTTGCGTTAACGTTAATCTTTA
CGATGGCGTTCAGCAACATGTCTGCGCAGGCTGCTGAAGAAGCAAAAGAAAAT
ATTTAATTGGCTTTAATGAGCAGGAAGCTGTCAGTGAGTTTGTAGAACAAGTAGA
GGCAAATGACGGCGTCGCCATTCTCTCTGAGGAAGAGGAAGTCGAAATTGAATT
GCTTCATGAATTTGAAACGATTCCTGTTTTATCCGTTGAGTTAAGCCCAGAAGAT
GTGGACGCGCTTGAACTCGATCCAGCGATTTCTTATATTGAAGAGGATGCAGAAG
TAACGACAATGGCGCAATCGGTACCATGGGGAATTAGCCGTGTGCAAGCCCCAG
CTGCCCATAACCGTGGATTGACAGGTTCTGGTGTAAAAGTTGCTGTCCTCGATAC
AGGTATTTCCACTCATCCAGACTTAAATATTCGTGGTGGCGCTAGCTTTGTACCA
GGGGAACCATCCACTCAAGATGGGAATGGGCATGGCACGCATGTGGCTGGGACG
ATTGCTGCTTTAAACAATTCGATTGGCGTTCTTGGCGTAGCACCGAACGCGGAAC
TATACGCTGTTAAAGTATTAGGGGCGAGCGGTATGGGTTCGGTCAGCTCGATTGC
CCAAGGATTGGAATGGGCAGGGAACAATGTTATGCACGTTGCTAATTTGAGTTTA
GGACTGCAGGCACCAAGTGCCACACTTGAGCAAGCTGTTAATAGCGCGACTTCT
AGAGGCGTTCTTGTTGTAGCGGCATCTGGCAATTCAGGTGCAGGCTCAATCAGCT
ATCCGGCCCGTTATGCGAACGCAATGGCAGTCGGAGCTACTGACCAAAACAACA
ACCGCGCCAGCTTTTCACAGTATGGCGCAGGGCTTGACATTGTCGCACCAGGTGT
AAACGTGCAGAGCACATACCCAGGTTCAACGTATGCCAGCTTAAACGGTACATC

GATGGCTACTCCTCATGTTGCAGGTGCAGCAGCCCTTGTTAAACAAAAGAACCCA
TCTTGGTCCAATGTCCAAATCCGCAATCATCTAAAGAATACGGCAACGAGCTTAG
GAAGCACGAACTTGTATGGAAGCGGACTTGTCAATGCAGAAGCGGCAACACGTT
AA (SEQ ID NO:20)

### Amino acid sequence of the ER11 subtilisin protease expressed from the ribosomal protein promoters

VRSKKLWISLLFALTLIFTMAFSNMSAQAAEEAKEKYLIGFNEQEAVSEFVEQVEAN
DGVAILSEEEEVEIELLHEFETIPVLSVELSPEDVDALELDPAISYIEEDAEVTTMAQSV
PWGISRVQAPAAHNRGLTGSGVKVAVLDTGISTHPDLNIRGGASFVPGEPSTQDGNG
HGTHVAGTIAALNNSIGVLGVAPNAELYAVKVLGASGMGSVSSIAQGLEWAGNNV
MHVANLSLGLQAPSATLEQAVNSATSRGVLVVAASGNSGAGSISYPARYANAMAV
GATDQNNNRASFSQYGAGLDIVAPGVNVQSTYPGSTYASLNGTSMATPHVAGAAA
LVKQKNPSWSNVQIRNHLKNTATSLGSTNLYGSGLVNAEAATR (SEQ ID NO:21)

The different strains constructed from the fusion of the promoters to the target genes are listed in Table 2-2 below.

| **Table 2-2: List of strains constructed** |
|---|
| BG8000 P*aprE*-FNA |
| BG8000 P*aprE*-FNA |
| BG8010 P*aprE*-FNA |
| BG8010 P*aprE*-FNA |
| BG8000 P*aprE*-ER11 |
| BG8000 P*aprE*-ER11 |
| BG8010 P*aprE*-ER11 |
| BG8010 P*aprE*-ER11 |
| BG8010 P*rpsD*-FNA |
| BG8010 P*rpsJ*-FNA |
| BG8010 P*rpsJ-*GFP |
| BG8010 P*rpsD-*GFP |
| BG8010 P*aprE*-GFP |

### Example 7: Cell density measurements of GFP and FNA expressing strains

To test for cell growth, one colony each of the constructed strains was inoculated in Luria Broth containing 25 µg/ml chloramphenicol for strains expressing from *aprE* or *rpoD* promoters and grown overnight at 30°C. One ml of each pre-culture was used to inoculate 32 ml of 2xSNB medium (see composition below) and grown at 37°C in shake flasks at 280 rpm, 70% humidity. At hourly intervals from 4 hours to 8 hours of growth, optical densities of each culture was measured at 600 nm using a SpectraMax reader. The cell density measurements of GFP and FNA expressing strains are shown in Figures 16 (GFP) and 17 (FNA). The growth of strains containing the different constructs was comparable.
2xSNB medium:
Stock solutions (filter sterilized): 25x SNB salts-CaCl₂*2H₂O (3.7 g/L), FeSO₄*7H₂O (9.6 mg/L), MnCl₂*4H₂O (6 mg/L), KCl (25 g/L), MgSO₄*7H₂O (3.26 g/L), Maltrin 150 10%. Prepare 500mL of 16 g/L solution of Difco Nutrient Broth, autoclave, add 20mL 25xSNB salts, and 25mL 10% Maltrin 150.

### Example 8: Protein expression of GFP and FNA from ribosomal promoters

The production of FNA or intracellular expression of GFP driven by the selected promoters was tested in BG8000 and BG8010 strains. The cells were grown as described for the cell density measurements in Example 7. At hourly intervals from 4 hours to 8 hours of growth, supernatants of cultures were analyzed for AAPF activity (subtilisin expression). GFP expression was measured as Relative Fluorescence Units (RFU) expressed in the cell.

The AAPF activity of a sample was measured as the rate of hydrolysis of N-succinyl-L-alanyl-L-alanyl-L-prolyl-L-phenyl-p-nitroanilide (suc-AAPF-pNA). The reagent solutions used were: 100 mM Tris/HCl, pH 8.6, containing 0.005% TWEEN®-80 (Tris dilution buffer and 160 mM suc-AAPF-pNA in DMSO (suc-AAPF-pNA stock solution) (Sigma: S-7388). To prepare a suc-AAPF-pNA working solution, 1 ml suc-AAPF-pNA stock solution was added to 100 ml Tris/ HCl buffer and mixed well for at least 10 seconds. The assay was performed by diluting the samples in the assay buffer (5 µl in 195 µl). Then, 180µl of assay buffer with AAPF substrate was added to 20 µl of the diluted sample arrayed in a microtiter plate. The solutions were mixed for 5 sec., and the absorbance change in kinetic mode (20 readings in 5 minutes) was read at 405 nm in a SpectraMax reader, at 25°C.

For measuring GFP expression in RFU, 150 µl of each culture sample was loaded into a microtiter plate and fluorescence measurements were taken using the SpectraMax reader using an excitation wavelength at 485 nm, emission wavelength at 508 nm, with a cutoff at 495 nm.

Expression of GFP and FNA from the different promoters is shown in Figures 18 (GFP) and 19 (FNA). Protein expression from non-amplified ribosomal protein promoter was higher than that seen from amplified *aprE* promoter.

### Example 9: Protein expression from SigmaA dependent promoter

As different levels of protein expression are observed from different promoters, this experiment compared FNA expression from amplified *rpo*D promoter (a promoter for the sigmaA housekeeping sigma factor in *B subtilis*) with that from amplified *apr*E promoter. BG8010 strains expressing FNA from *rpo*D and *apr*E were amplified using 25µg/mL chloramphenicol. Cell density measurements and FNA expression was studied as described in Examples 7 and 8 respectively. Results are shown in Figures 20 and 21. Cell growth from all strains was comparable.

## Claims

1. An isolated nucleic acid comprising a *B. subtilis* ribosomal RNA promoter operably linked to a nucleic acid encoding a protein of interest,
wherein the protein of interest is selected from the group consisting of a protease, cellulase, amylase, xylanase, phytase, mannanase, hemicellulase, carbohydrase, hydrolase, esterase, oxidase, permease, pullulanase, laccase, lipase, reductase, isomerase, epimerase, tautomerase, transferase, kinase or phosphatase,
wherein the ribosomal RNA promoter comprises the nucleotide sequence of any one of SEQ ID NOs: 2&3, a nucleic acid that is a subsequence having at least 80% sequence identity to any one of SEQ ID NOs: 2&3 that retains promoter activity or combinations thereof.

2. The isolated nucleic acid of claim 1, wherein the nucleic acid comprises the nucleotide sequence of SEQ ID NO: 3.

3. The isolated nucleic acid of any of the preceding claims, wherein the protein of interest is a protease.

4. The isolated nucleic acid of claim 3, wherein the protease is subtilisin.

5. The isolated nucleic acid of claim 3, wherein the protease is encoded by SEQ ID NO: 9, 11, 18, or 20.

6. An expression vector comprising the nucleic acid of any one of the preceding claims.

7. A microorganism comprising the expression vector of claim 6.

8. A method for producing a protein of interest, comprising culturing the microorganism of claim 7 under conditions suitable for the microorganism to produce the protein and optionally recovering the protein of interest thus produced.

## Patentansprüche

1. Isolierte Nucleinsäure, umfassend einen ribosomalen RNA-Promotor von *B. subtilis,* der wirkfähig an eine Nucleinsäure gebunden ist, die ein Protein von Interesse codiert,
wobei das Protein von Interesse ausgewählt ist aus der Gruppe bestehend aus Protease, Cellulase, Amylase, Xylanase, Phytase, Mannanase, Hemicellulase, Carbohydrase, Hydrolase, Esterase, Oxidase, Permease, Pullulanase, Laccase, Lipase, Reduktase, Isomerase, Epimerase, Tautomerase, Transferase, Kinase oder Phosphatase,
wobei der ribosomale RNA-Promotor die Nucleotidsequenz einer der SEQ ID NO: 2 & 3, eine Nukleinsäure, die eine Untersequenz mit mindestens 80% Sequenzidentität zu einer der SEQ ID NO: 2 & 3 ist und die die Promotoraktivität beibehält, oder Kombinationen davon umfasst.

2. Isolierte Nucleinsäure nach Anspruch 1, wobei die Nucleinsäure die Nucleotidsequenz von SEQ ID NO: 3 umfasst.

3. Isolierte Nucleinsäure nach einem der vorhergehenden Ansprüche, wobei das Protein von Interesse eine Protease ist.

4. Isolierte Nucleinsäure nach Anspruch 3, wobei die Protease Subtilisin ist.

5. Isolierte Nucleinsäure nach Anspruch 3, wobei die Protease durch SEQ ID NO: 9, 11, 18 oder 20 codiert ist.

6. Expressionsvektor umfassend die Nucleinsäure nach einem der vorhergehenden Ansprüche.

7. Mikroorganismus, der den Expressionsvektor nach Anspruch 6 umfasst.

8. Verfahren zum Herstellen eines Proteins von Interesse, umfassend das Kultivieren des Mikroorganismus nach Anspruch 7 unter Bedingungen, die für den Mikroorganismus zur Herstellung des Proteins geeignet sind, und wahlweise zum Gewinnen des so hergestellten Proteins von Interesse.

## Revendications

1. Acide nucléique isolé comprenant un promoteur ARN ribosomal B. subtilis qui est fonctionnellement lié à un acide nucléique encodant une protéine d'intérêt,
dans lequel la protéine d'intérêt est sélectionnée dans le groupe constitué d'une protéase, d'une cellulase, d'une amylase, d'une xylanase, d'une phytase, d'une mannanase, d'une hemicellulase, d'une carbohydrase, d'une hydrolase, d'une estérase, d'une oxidase, d'une perméase, d'une pullulanase, d'une laccase, d'une lipase, d'une réductase, d'une isomérase, d'une épimérase, d'une tautomérase, d'une transférase, d'une kinase ou d'une phosphatase,
dans lequel le promoteur ARN ribosomal comprend la séquence de nucléotide de l'une quelconque des SEQ ID Nos : 2 et 3, un acide nucléique qui est une subséquence avec au moins 80% d'identité de séquence avec l'une quelconque des SEQ ID Nos : 2 et 3 qui conserve l'activité de promoteur ou des combinaisons de celles-ci.

2. Acide nucléique isolé selon la revendication 1, dans lequel l'acide nucléique comprend la séquence de nucléotide de SEQ ID No :3.

3. Acide nucléique isolé selon l'une quelconque des revendications précédentes, dans lequel la protéine d'intérêt est une protéase.

4. Acide nucléique isolé selon la revendication 3, dans lequel la protéase est la subtilisine.

5. Acide nucléique isolé selon la revendication 3, dans lequel la protéase est encodée par les SEQ ID Nos : 9, 11, 18 ou 20.

6. Vecteur d'expression, comprenant l'acide nucléique selon l'une quelconque des revendications précédentes.

7. Microorganisme, comprenant le vecteur d'expression selon la revendication 6.

8. Procédé pour produire une protéine d'intérêt, comprenant la cultivation du microorganisme selon la revendication 7 sous des conditions qui sont appropriées pour que le microorganisme produise la protéine et optionnellement recouvre la protéine d'intérêt ainsi produite.
